# EUROPEAN PATENT APPLICATION

(11) **EP 3 360 573 A1**
(43) Date of publication of application: **15.08.2018**
(21) Application number: 16853615.9
(22) Date of filing: 05.10.2016
(51) Int. Cl.: A61K 45/00, A61K 31/18, A61K 31/381, A61K 31/44, A61K 45/06, A61P 13/12

(54) **MEDICINAL DRUG FOR PREVENTING OR INHIBITING ACUTE KIDNEY INJURY**

(30) Priority: 05.10.2015 JP 2015197921; 24.12.2015 JP 2015252659
(71) Applicant: National Cerebral and Cardiovascular Center, Suita-shi Osaka 565-8565 (JP)
(72) Inventor: KANGAWA, Kenji, Suita-shi Osaka 565-8565 (JP); HOSODA, Hiroshi, Suita-shi Osaka 565-8565 (JP); NOJIRI, Takashi, Suita-shi Osaka 565-8565 (JP)
(74) Representative: Witte, Weller & Partner Patentanwälte mbB
(86) International application number: PCT/JP2016/079606
(87) International publication number: WO 2017/061461

(57) **Abstract**

The present invention provides a novel medicament for preventing or inhibiting acute kidney injury, the medicament comprising, as an active ingredient, an angiotensin II receptor type 2 agonist or a pharmacologically acceptable salt thereof, and being used for treatment or prevention of acute kidney injury. Also, the present invention provides a novel method for preventing or inhibiting acute kidney injury, the method comprising administering, to a patient, an effective amount of an angiotensin II receptor type 2 agonist or a pharmacologically acceptable salt thereof in combination with an anticancer agent and/or an antitumor agent.

## Description

### TECHNICAL FIELD

The present invention relates to a novel medicinal composition for preventing or inhibiting acute kidney injury, the composition comprising, as an active ingredient, an angiotensin II receptor type 2 (hereinafter referred to as AT₂ receptor) agonist or a pharmacologically acceptable salt thereof. In addition, the present invention relates to a novel method for preventing or inhibiting acute kidney injury.

### BACKGROUND ART

Renal failure is a condition in which the normal renal function mainly based on filtration is impaired, and broadly divided into acute renal failure (ARF) and chronic renal failure (CRF) depending on the speed of loss of the renal function.

Chronic renal failure is a clinical condition in which the renal function is gradually impaired over a long period of time due to hypertension, diabetes, etc. In the state of chronic renal failure, recovery of the renal function cannot be expected. There are no effective therapeutic methods for chronic renal failure, and treatment mainly consists of delaying the progress and preventing complications. Terminal renal failure advanced from chronic renal failure requires dialysis or renal transplantation.

In contrast, acute renal failure is a clinical condition in which abrupt loss of the renal function causes several symptoms, such as azotemia, electrolyte imbalance, and uremia, and in serious cases, where life is threatened, dialysis is introduced. In acute renal failure, unlike chronic renal failure, removal of the cause may lead to recovery of the renal function, and therefore, early detection is important to prevent increase in severity.

Various definitions have been used for acute renal failure, and there have been no consensus diagnostic criteria. Recently, however, to integrate the conventional concepts and to diagnose a renal disorder or renal function deterioration at early stages, the concept of acute kidney injury (AKI) has been proposed. In the AKIN criteria proposed as diagnostic classification for acute kidney injury, (1) an increase in serum creatinine of 0.3 mg/dL or more within 48 hours; or (2) an increase in serum creatinine of 50% or more within 48 hours; or (3) urine output of 0.5 mL/kg or less per hour continuing for 6 hours or more is defined as acute kidney injury (Non Patent Literature 1).

Acute kidney injury results from various factors. In particular, administration of a platinum-based antitumor agent (cisplatin etc.), which is a main therapeutic agent commonly used in cancer chemotherapy, may damage epithelial cells of uriniferous tubules and may cause acute kidney injury and other complications as a side effect. In particular, cisplatin-induced kidney injury increases in a dose-dependent manner, and therefore, at the onset of kidney injury, the administration of cisplatin must be restricted. For this reason, in the current clinical practice, for the purpose of preventing or inhibiting the onset of acute kidney injury, a large amount of physiological saline is administered or an electrolyte solution is infused before and/or after the administration of cisplatin, but it is virtually impossible to completely prevent or inhibit acute kidney injury.

In a living body, angiotensinogen is converted to angiotensin I by renin, and angiotensin I is then converted, by converting enzymes, such as angiotensin-converting enzyme (ACE), to angiotensin II (AngII), which has strong and various physiological actions.

As receptors to which AngII binds, angiotensin II receptor type 1 (hereinafter referred to as AT₁ receptor) and AT₂ receptor have been identified. Conventionally well known actions of AngII, such as vasopressor and vasoconstrictive actions, have been understood to be mediated mainly by the classic AT₁ receptor. Meanwhile, the AT₂ receptor has recently been identified to have an antagonistic action on the AT₁ receptor in many types of cells and tissues and have various functions, including hypotension, cell growth inhibition, hypertrophy inhibition, apoptosis promotion, extracellular matrix production inhibition, etc. These functions mostly act to inhibit the onset and the progress of pathological conditions. The AT₂ receptor is extensively and highly expressed in the fetal period, but the expression level rapidly decreases after birth. However, tissue-specific re-expression under pathological conditions, such as angiopathy and cardiovascular remodeling after myocardial infarction, has come to be known, and the importance of the AT₂ receptor relating to the inhibition of the onset and the progress of various diseases has attracted attention.

Predictable general pharmacological actions of activated AT₂ receptors are reported also by de Gasparo et al. (Non Patent Literature 2), and AT₂ receptor agonists are promising for use as a medicament having therapeutic or preventive effects on various diseases. Examples of the target disease include many diseases involving the renin-angiotensin-aldosterone system (hereinafter referred to as RAAS), for example, metabolic and cardiovascular diseases, such as cerebral infarction, a kidney disease, a cardiac disease, hypertension, diabetes, metabolic syndrome, etc.

As non-peptidic AT₂ receptor agonists, 3-phenyl-2-thiophenesulfonamide and biphenylsulfonamide compounds have been disclosed so far (Non Patent Literature 3 and 4, Patent Literature 1 to 9). The compounds shown in the Non Patent Literature or Patent Literature have a common characteristic, i.e., a combination of a bisaryl structure and a sulfonamide group, etc.

Compound 21 (C21) having a 3-phenyl-2-thiophenesulfonamide structure and an AT₂ receptor agonistic action exhibits anti-inflammatory action, and has a therapeutic effect on the kidney (Non Patent Literature 5). Also described is that when LPS as an endotoxin was allowed to act on human renal cells, treatment with C21 lowered the levels of TNF-α and IL-6 (Non Patent Literature 6). In addition, AT₂ receptor deficiency aggravates renal injury in chronic renal failure model mice, suggesting that activation of AT₂ receptors is useful for the treatment of chronic renal failure (Non Patent Literature 7) . However, none of these documents describe the relation between AT₂ receptors and acute kidney injury.

Sulfonyl malonamide derivatives are also known as AT₂ receptor agonists, and used for the treatment or prevention of kidney diseases etc. (Patent Literature 10), but there is no disclosure regarding the prevention or inhibition of acute kidney injury.

Further, sulfonyl malonamide derivatives are also known as herbicides (Patent Literature 11), but disclosed are unsubstituted sulfonyl malonamide derivatives and nothing is mentioned regarding a sulfonyl malonamide derivative having a substitution at position 2 of the malonic acid structure, which derivative serves as an AT₂ receptor agonist.

### CITATION LIST

### Patent Literature

Patent Literature 1: WO 02/096883
Patent Literature 2: WO 06/109058
Patent Literature 3: WO 03/064414
Patent Literature 4: WO 04/046128
Patent Literature 5: WO 04/046137
Patent Literature 6: WO 04/085420
Patent Literature 7: WO 06/109056
Patent Literature 8: WO 06/109058
Patent Literature 9: WO 04/046141
Patent Literature 10: WO 2008/156142
Patent Literature 11: IN Pat. No. 178290

### Non Patent Literature

Non Patent Literature 1: Critical Care 2008, 12: R110
Non Patent Literature 2: Pharmacol. Rev., 52, 415-472 (2000)
Non Patent Literature 3: J. Med. Chem., 47, 5995-6008 (2004)
Non Patent Literature 4: J. Med. Chem., 49, 7160-7168 (2006)
Non Patent Literature 5: Journal of Hypertension, 2009, 27 (12), 2444-2451
Non Patent Literature 6: Hypertension 61; 1218-1226 (2013)
Non Patent Literature 7: Kidney International 2009, 75, 1039-1049

### SUMMARY OF INVENTION

### TECHNICAL PROBLEM

A major objective of the present invention is to provide a novel medicinal composition etc. for preventing or inhibiting acute kidney injury, in particular, acute kidney injury induced by an anticancer agent and/or an antitumor agent. Another objective of the present invention is to provide a novel method for preventing or inhibiting acute kidney injury, in particular, acute kidney injury induced by an anticancer agent and/or an antitumor agent.

### SOLUTION TO PROBLEM

In order to achieve the above objectives, the present inventors conducted intensive investigations and found that an AT₂ receptor ligand, in particular, an AT₂ receptor agonist acts on an AT₂ receptor and thereby effectively prevents or inhibits acute kidney injury. Based on the finding, they carried out further investigations and completed the present invention.

That is, the present invention relates to (1) a medicament for preventing or inhibiting acute kidney injury, the medicament comprising, as an active ingredient, an AT₂ receptor agonist or a pharmacologically acceptable salt thereof.

Also, the present invention relates to (2) the medicament according to the above (1), wherein the acute kidney injury is acute kidney injury induced by an anticancer agent and/or an antitumor agent.

Also, the present invention relates to (3) the medicament according to the above (2), wherein the anticancer agent and/or the antitumor agent is a platinum-based antitumor agent.

Also, the present invention relates to (4) the medicament according to any one of the above (1) to (3), wherein the AT₂ receptor agonist is a sulfonyl malonamide derivative represented by the following general formula (I): 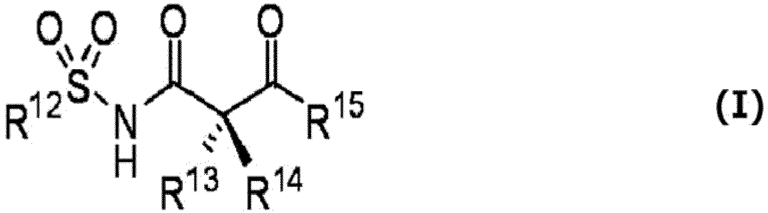 [wherein
R¹² denotes 2-naphthyl, trans-β-styryl, phenethyl, 3-phenoxypropyl, or 4-phenylbutyl;
either of R¹³ and R¹⁴ denotes a hydrogen atom, and the other denotes isopropyl, isobutyl, neopentyl, allyl, -CH₂-R¹⁶ {wherein R¹⁶ denotes optionally substituted C₃₋₁₀ cycloalkyl, an optionally substituted heterocycle, or -CO-NR⁵R⁶ (wherein R⁵ and R⁶ may be the same or different and each represent a hydrogen atom, C₁₋₆ alkyl, optionally substituted aryl, or optionally substituted heteroaryl, or R⁵ and R⁶ may form, together with the nitrogen atom to which they are bonded, optionally substituted cyclic amino)}, -(CH₂)₂-R^{16'} (wherein R^{16'} denotes cyano or C₁₋₆ alkoxy), or -(CH₂)ₙ-Ar² (wherein n denotes an integer of 1 to 3, and Ar² denotes substituted phenyl or optionally substituted heteroaryl), or R¹³ and R¹⁴ may form, together with the carbon atom to which they are bonded, a moiety represented by the following formula: 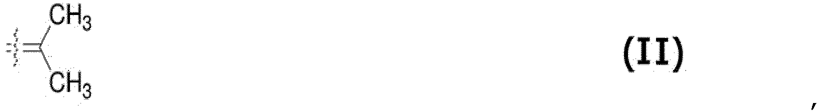 and
R¹⁵ denotes di(C₁₋₆ alkyl)amino or a moiety represented by the following formula: 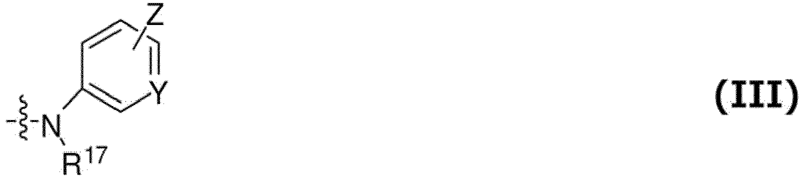 (wherein Z denotes a hydrogen atom, a halogen atom, or trifluoromethyl, Y denotes a nitrogen atom or CH, and R¹⁷ denotes ethyl, isopropyl, or 3-pentyl, with the proviso that when Y is a nitrogen atom, Z denotes a hydrogen atom)], or a pharmacologically acceptable salt thereof.

Also, the present invention relates to (5) the medicament according to the above (4), wherein the angiotensin II receptor type 2 agonist is a sulfonyl malonamide derivative selected from N,N-diethyl-2-{4-[(2,6-difluorobenzoyl)amino]benzyl}-N'-(2-naphthylsulfonyl)malonamide,
(2S)-2-[4-(benzoylamino)benzyl]-N,N-diethyl-N'-(2-naphthyls ulfonyl)malonamide,
(2S)-N,N-diethyl-2-{4-[(2-fluorobenzoyl)amino]benzyl}-N'-(2 -naphthylsulfonyl)malonamide,
(2S)-N,N-diethyl-2-{4-[(3-fluorobenzoyl)amino]benzyl}-N'-(2 -naphthylsulfonyl)malonamide,
(2S)-N,N-diethyl-2-{4-[(2,4-difluorobenzoyl)amino]benzyl}-N '-(2-naphthylsulfonyl)malonamide,
(2S)-N,N-diethyl-2-{4-[(4-methylbenzoyl)amino]benzyl}-N'-(2 -naphthylsulfonyl)malonamide,
(2S)-N,N-diethyl-N'-(2-naphthylsulfonyl)-2-{4-[(2-thienoyl) amino]benzyl}malonamide,
(2S)-N,N-diethyl-2-{4-[(2-furoyl)amino]benzyl}-N'-(2-naphth ylsulfonyl)malonamide,
(2S)-2-{4-[(2-amino-5-fluorobenzoyl)amino]benzyl}-N,N-dieth yl-N'-(2-naphthylsulfonyl)malonamide,
(2S)-2-{4-[(2-amino-6-fluorobenzoyl)amino]benzyl}-N,N-dieth yl-N'-(2-naphthylsulfonyl)malonamide,
(2S)-N,N-diethyl-N'-(2-naphthylsulfonyl)-2-{4-[(2-pyridylca rbonyl)amino]benzyl}malonamide,
(2S)-2-{4-[(2-amino-4-chlorobenzoyl)amino]benzyl}-N,N-dieth yl-N'-(2-naphthylsulfonyl)malonamide,
(2S)-2-{4-[(2-aminobenzoyl)amino]benzyl}-N,N-diethyl-N'-(2-naphthylsulfonyl)malonamide,
(2S)-2-{4-[(2-amino-5-chlorobenzoyl)amino]benzyl}-N,N-dieth yl-N'-(2-naphthylsulfonyl)malonamide,
(2S)-2-{4-[(2-amino-4,5-difluorobenzoyl)amino]benzyl}-N,N-d iethyl-N'-(2-naphthylsulfonyl)malonamide,
(2S)-2-{4-[(2-amino-4-fluorobenzoyl)amino]benzyl}-N,N-dieth yl-N'-(2-naphthylsulfonyl)malonamide,
(2S)-2-{4-[(2-amino-5-methylbenzoyl)amino]benzyl}-N,N-dieth yl-N'-(2-naphthylsulfonyl)malonamide,
2-(4-fluorobenzyl)-N-isopropyl-N-(3-pyridyl)-N'-((E)-styryl sulfonyl)malonamide,
2-allyl-N-(4-fluorophenyl)-N-isopropyl-N'-((E)-styrylsulfon yl)malonamide,
N-(4-fluorophenyl)-2-isobutyl-N-isopropyl-N'-((E)-styrylsul fonyl)malonamide,
N-(4-fluorophenyl)-2-isobutyl-N-isopropyl-N'-phenethylsulfo nyl malonamide,
N-(4-fluorophenyl)-2-isobutyl-N-isopropyl-N'-(2-naphthylsul fonyl)malonamide,
(2S or 2R)-2-cyclopropylmethyl-N-(4-fluorophenyl)-N-isopropyl-N'-( (E)-2-styrylsulfonyl)malonamide,
2-cyclopropylmethyl-N-(4-fluorophenyl)-N-isopropyl-N'-phene thylsulfonyl malonamide, and
2-cyclopropylmethyl-N-(4-fluorophenyl)-N-isopropyl-N'-(2-na phthylsulfonyl)malonamide,
or a pharmacologically acceptable salt thereof.
(6) The present invention relates to a combination medicament of an AT₂ receptor agonist or a pharmacologically acceptable salt thereof and an anticancer agent and/or an antitumor agent.
(7) The present invention relates to a medicinal composition for preventing or inhibiting acute kidney injury, the medicinal composition comprising, as active ingredients, an AT₂ receptor agonist or a pharmacologically acceptable salt thereof and an anticancer agent and/or an antitumor agent.
(8) The present invention relates to a medicinal composition for preventing or inhibiting acute kidney injury, the medicinal composition comprising an AT₂ receptor agonist or a pharmacologically acceptable salt thereof, which composition is used in combination with an anticancer agent and/or an antitumor agent.
(9) The present invention relates to a method for preventing or inhibiting acute kidney injury, the method comprising administering, to a patient, an effective amount of an AT₂ receptor agonist or a pharmacologically acceptable salt thereof in combination with an anticancer agent and/or an antitumor agent.
(10) The present invention relates to a method for preventing or inhibiting acute kidney injury, the method comprising administering, to a patient, an effective amount of an AT₂ receptor agonist or a pharmacologically acceptable salt thereof.
(11) The present invention relates to the method according to the above (10), wherein the acute kidney injury is acute kidney injury induced by an anticancer agent and/or an antitumor agent.
(12) The present invention relates to an AT₂ receptor agonist or a pharmacologically acceptable salt thereof for use in preventing or inhibiting acute kidney injury.
(13) The present invention relates to the angiotensin II receptor type 2 agonist or a pharmacologically acceptable salt thereof for use according to the above (12), wherein the acute kidney injury is acute kidney injury induced by an anticancer agent and/or an antitumor agent.
(14) The present invention relates to use of an AT₂ receptor agonist or a pharmacologically acceptable salt thereof for producing a medicament for preventing or inhibiting acute kidney injury.
(15) The present invention relates to the use according to the above (14), wherein the acute kidney injury is acute kidney injury induced by an anticancer agent and/or an antitumor agent.
(16) The present invention relates to use of an AT₂ receptor agonist or a pharmacologically acceptable salt thereof for preventing or inhibiting acute kidney injury.
(17) The present invention relates to the use according to the above (16), wherein the acute kidney injury is acute kidney injury induced by an anticancer agent and/or an antitumor agent.

### ADVANTAGEOUS EFFECTS OF INVENTION

When acute kidney injury appears due to the use of an anticancer agent and/or an antitumor agent (in particular cisplatin), reduction of the dosage, change to another antitumor agent, or the like is required. Therefore, in clinical practice, treatment must be based on the balance among the antitumor effect, side effects, and safety.

The medicament, the prevention or inhibition method, etc. of the present invention have an excellent effect of effectively preventing or inhibiting acute kidney injury, in particular, acute kidney injury induced by an anticancer agent and/or an antitumor agent, and metabolites thereof. In particular, the medicament, the prevention or inhibition method, etc. of the present invention effectively prevent or inhibit acute kidney injury by the action of an AT₂ receptor agonist.

The medicament of the present invention administered before the administration of an anticancer agent and/or an antitumor agent is promising for inhibiting acute kidney injury without the use of a large amount of an infusion solution etc., and for allowing for continuous administration of an anticancer agent and/or an antitumor agent having an excellent therapeutic effect without reduction of the dosage.

Therefore, the use of the medicament, the prevention or inhibition method, etc. of the present invention achieves cancer treatment in which the effect of an anticancer agent and/or an antitumor agent can be fully exerted and the risk of causing acute kidney injury is avoided.

### BRIEF DESCRIPTION OF DRAWINGS

Fig. 1 shows a graph showing the urinary albumin/creatinine ratio at 72 hours from the administration of cisplatin (CDDP) in the cases of the injection of CDDP and/or the oral ingestion of Compounds A to C in a CDDP induced acute kidney injury inhibition test.
Fig. 2 shows a graph showing the blood urea nitrogen (BUN) at 72 hours from the administration of cisplatin (CDDP) in the cases of the injection of CDDP and/or the oral ingestion of Compounds A to C in a CDDP induced acute kidney injury inhibition test.
Fig. 3 shows a graph showing the urinary albumin/creatinine ratio at 72 hours from the administration of cisplatin (CDDP) in the cases of the injection of CDDP and/or the oral ingestion of Compound C in a CDDP induced acute kidney injury inhibition test using AT₂ receptor-deficient mice.
Fig. 4 shows a graph showing the blood urea nitrogen (BUN) at 72 hours from the administration of cisplatin (CDDP) in the cases of the injection of CDDP and/or the oral ingestion of Compound C in a CDDP induced acute kidney injury inhibition test using AT₂ receptor-deficient mice.

### DESCRIPTION OF EMBODIMENTS

Examples of the AT₂ receptor agonist of the present invention include the compounds described in WO 2008/156142, WO 2002/096883, etc., and preferred is the following compound (I) described in WO 2008/156142:
a sulfonyl malonamide derivative represented by the following general formula (I): 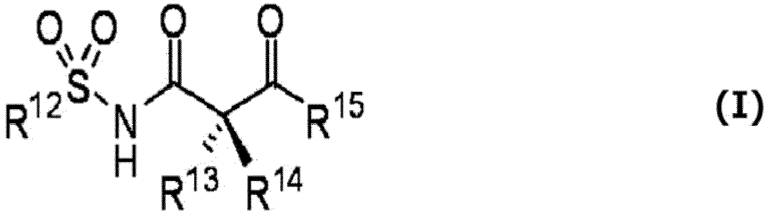 [wherein
R¹² denotes 2-naphthyl, trans-β-styryl, phenethyl, 3-phenoxypropyl, or 4-phenylbutyl;
either of R¹³ and R¹⁴ denotes a hydrogen atom, and the other denotes isopropyl, isobutyl, neopentyl, allyl, -CH₂-R¹⁶ {wherein R¹⁶ denotes optionally substituted C₃₋₁₀ cycloalkyl, an optionally substituted heterocycle, or -CO-NR⁵R⁶ (wherein R⁵ and R⁶ may be the same or different and each represent a hydrogen atom, C₁₋₆ alkyl, optionally substituted aryl, or optionally substituted heteroaryl, or R⁵ and R⁶ may form, together with the nitrogen atom to which they are bonded, optionally substituted cyclic amino)}, -(CH₂)₂-R^{16'} (wherein R^{16'} denotes cyano or C₁₋₆ alkoxy), or -(CH₂)ₙ-Ar² (wherein n denotes an integer of 1 to 3, and Ar² denotes substituted phenyl or optionally substituted heteroaryl), or R¹³ and R¹⁴ may form, together with the carbon atom to which they are bonded, a moiety represented by the following formula: 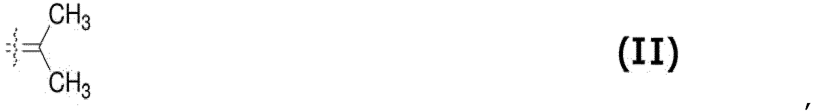 and
R¹⁵ denotes di(C₁₋₆ alkyl)amino or a moiety represented by the following formula: 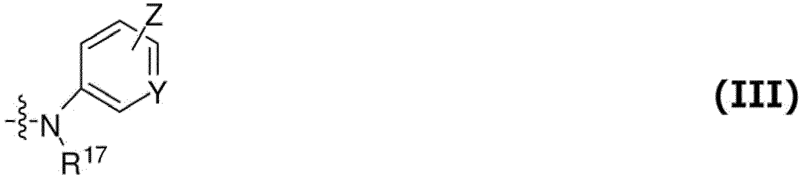 (wherein Z denotes a hydrogen atom, a halogen atom, or trifluoromethyl, Y denotes a nitrogen atom or CH, and R¹⁷ denotes ethyl, isopropyl, or 3-pentyl, with the proviso that when Y is a nitrogen atom, Z denotes a hydrogen atom)], or a pharmacologically acceptable salt thereof.

Hereinafter, each of the substituents in the above general formula (I) will be described in detail.

The C₁₋₆ alkyl means a linear or branched hydrocarbon group having 1 to 6 carbon atoms, and examples thereof include methyl, ethyl, propyl, isopropyl, butyl, isobutyl, sec-butyl, tert-butyl, pentyl, isopentyl, neopentyl, hexyl, isohexyl, etc.

The C₂₋₆ alkenyl means a linear or branched unsaturated hydrocarbon group having 2 to 6 carbon atoms and one or more carbon-carbon double bonds, and examples thereof include vinyl, allyl, 1-propenyl, isopropenyl, 2-butenyl, 1,3-butadienyl, 2-methyl-2-propenyl, prenyl, isopentenyl, 2-hexenyl, etc.

The C₂₋₆ alkynyl means a linear or branched unsaturated hydrocarbon group having 2 to 6 carbon atoms and one or more carbon-carbon triple bonds, and examples thereof include ethynyl, 2-propynyl, 2-butynyl, 3-butynyl, 3-pentynyl, 5-hexynyl, etc.

The C₁₋₆ alkoxy has the same meaning as the above "C₁₋₆ alkyl", and examples thereof include methoxy, ethoxy, propoxy, isopropoxy, butoxy, pentyloxy, hexyloxy, etc.

The C₃₋₁₀ cycloalkyl means a saturated cyclic hydrocarbon group having 3 to 10 carbon atoms, and examples thereof include cyclopropyl, cyclobutyl, cyclopentyl, cyclohexyl, cycloheptyl, etc. These cycloalkyls may be condensed with a benzene ring to form indane (for example, indan-1-yl, indan-2-yl, etc.), tetrahydronaphthalene (for example, tetrahydronaphthalen-5-yl, tetrahydronaphthalen-6-yl, etc.), etc.

The C₃₋₁₀ cycloalkyl C₁₋₆ alkyl means the above "C₁₋₆ alkyl" substituted with the above "C₃₋₁₀ cycloalkyl". Preferred are those where the "C₁₋₆ alkyl" is an alkyl having 1 to 3 carbon atoms, and examples thereof include cyclopropylmethyl, 2-cyclobutylethyl, 3-cyclopentylpropyl, cyclohexylmethyl, 2-cyclohexylethyl, cycloheptylmethyl, etc.

The aryl preferably means an aromatic hydrocarbon group having 6 to 14 carbon atoms, and examples thereof include phenyl, naphthyl, etc. The group encompasses ortho-fused bicyclic groups having 8 to 10 ring atoms in which at least one ring is an aromatic ring (for example, indenyl etc.), etc.

The aryl C₁₋₆ alkyl means the above "C₁₋₆ alkyl" substituted with the above "aryl", and examples thereof include benzyl, benzhydryl, phenethyl, 1-phenylethyl, 3-phenylpropyl, 4-phenylbutyl, 5-phenylpentyl, 6-phenylhexyl, 1-naphthylmethyl, 2-naphthylmethyl, 2-(1-naphthyl)ethyl, 2-(2-naphthyl)ethyl, 3-(2-naphthyl)propyl, 4-(2-naphthyl)butyl, etc.

The aryl C₂₋₆ alkenyl means the above "C₂₋₆ alkenyl" substituted with the above "aryl". Preferred are those where the "C₂₋₆ alkenyl" is an alkenyl having 2 to 4 carbon atoms, and examples thereof include trans-β-styryl, cinnamyl, 3-(1-naphthyl)-2-propenyl, 3-(2-naphthyl)-2-propenyl, etc.

The heteroaryl means an aromatic group having, in addition to carbon atoms, one or more (preferably 1 to 4) hetero atoms selected from oxygen, sulfur, and/or nitrogen atoms. The group encompasses 5- or 6-membered monocyclic groups or ortho-fused bicyclic groups having 8 to 10 ring atoms derived from the monocyclic groups (in particular, benzo derivatives), derivatives obtained by fusing propenylene, trimethylene, or tetramethylene the monocyclic groups, and stable N-oxides thereof, etc. Examples thereof include pyrrolyl, furyl, thienyl, oxazolyl, isoxazolyl, imidazolyl, thiazolyl, isothiazolyl, pyrazolyl, triazolyl, tetrazolyl, 1,3,5-oxadiazolyl, 1,2,4-oxadiazolyl, 1,3,4-oxadiazolyl, 1,2,4-thiadiazolyl, pyridyl (2-pyridyl, 3-pyridyl, 4-pyridyl), pyrazinyl, pyrimidinyl, pyridazinyl, 1,2,4-triazinyl, 1,2,3-triazinyl, 1,3,5-triazinyl, benzoxazolyl, benzisoxazolyl, benzothiazolyl, benzisothiazolyl, benzimidazolyl, oxazolopyridyl, imidazopyridazinyl, thianaphthenyl, isothianaphthenyl, benzofuranyl, isobenzofuranyl, benzothienyl, chromenyl, isoindolyl, indolyl, indazolyl, isoquinolyl, quinolyl, phthalazinyl, quinoxalinyl, quinazolinyl, cinnolinyl, 2,1,3-benzoxadiazolyl, benzoxazinyl, pteridinyl, etc.

The heteroaryl C₁₋₆ alkyl means the above "C₁₋₆ alkyl" substituted with the above "heteroaryl". Preferred are those where the "C₁₋₆ alkyl" is an alkyl having 1 to 5 carbon atoms, and examples thereof include 2-pyridylmethyl, 3-pyridylmethyl, 4-pyridylmethyl, 2-(2-pyridyl)ethyl, 2-(3-pyridyl)ethyl, 2-(4-pyridyl)ethyl, 3-(2-pyridyl)propyl, 3-(3-pyridyl)propyl, 3-(4-pyridyl)propyl, 2-thienylmethyl, 3-thienylmethyl, 2-(2-thienyl)ethyl, 3-(2-thienyl)propyl, 4-pyrazolylmethyl, 2-(4-pyrazolyl)ethyl, 3-(4-pyrazolyl)propyl, 2-thiazolylmethyl, 4-thiazolylmethyl, 5-thiazolylmethyl, 2-(2-thiazolyl)ethyl, 3-(2-thiazolyl)propyl, 2-(4-thiazolyl)ethyl, 3-(4-thiazolyl)propyl, 2-(5-thiazolyl)ethyl, 3-(5-thiazolyl)propyl, 2-oxazolylmethyl, 4-oxazolylmethyl, 5-oxazolylmethyl, 2-(2-oxazolyl)ethyl, 3-(2-oxazolyl)propyl, 2-(4-oxazolyl)ethyl, 3-(4-oxazolyl)propyl, 2-(5-oxazolyl)ethyl, 3-(5-oxazolyl)propyl, 4-(1,2,3-triazolyl)methyl, 5-tetrazolylmethyl, 2-(5-tetrazolyl)ethyl, 1-imidazolylmethyl, 2-(1-imidazolyl)ethyl, 6-benzoxazolylmethyl, 1-benzimidazolylmethyl, etc.

The heterocycle means a cyclic hydrocarbon group having 1 to 3 hetero atoms selected from nitrogen, oxygen, sulfur atoms and/or the like. The group is non-aromatic, and may be saturated or partially unsaturated. The group encompasses not only monocycles but also spiro rings, and preferred are 4- to 7-membered monocyclic groups and 10- or 11-membered spirocyclic groups. Examples thereof include azetidinyl, pyrrolidinyl, piperidinyl, piperadinyl, morpholino, 1,4-diazepanyl, 1,2,5,6-tetrahydropyridyl, tetrahydropyranyl, cyclopentanespiro-4'-piperidinyl, etc.

The heterocycle may also have an aromatic ring condensed thereto. Examples of the condensed ring include indolinyl, isoindolinyl, 1,2,3,4-tetrahydroquinolyl, 1,2,3,4-tetrahydroisoquinolyl, spiro[indane-1,4'-piperidine]-1'-yl, etc.

The cyclic amino means a cyclic hydrocarbon group having at least one nitrogen atom via which the group is bonded. The ring may contain, in addition to the above-mentioned nitrogen atom, the same or different 1 to 3 hetero atoms selected from nitrogen, oxygen, and sulfur atoms, for example. The group is non-aromatic, and may be saturated or partially unsaturated. The group encompasses not only monocycles but also spiro rings, and preferred are 4- to 7-membered monocyclic groups and 10- or 11-membered spirocyclic groups. Examples thereof include azetidino, pyrrolidino, piperidino, piperadino, morpholino, 1,4-diazepan-1-yl, 1,2,5,6-tetrahydropyridino, tetrahydroimidazolino, cyclopentanespiro-4'-piperidino, etc.

The cyclic amino may also have an aromatic ring condensed thereto. Examples of the condensed ring include indolino, isoindolino, 1,2,3,4-tetrahydroquinolino, 1,2,3,4-tetrahydroisoquinolino, spiro[indane-1,4'-piperidine]-1'-yl, etc.

Examples of the halogen atom include a chlorine atom, a bromine atom, a fluorine atom, and an iodine atom.

The substituents in the "optionally substituted C₃₋₁₀ cycloalkyl", the "optionally substituted heterocycle", the "optionally substituted aryl", the "optionally substituted heteroaryl", and the "optionally substituted cyclic amino" may be 1 to 3 substituents selected from the substituent group A shown below. When two or more substituents are present, they may be the same or different.

Substituent group A: a halogen atom (as defined above), hydroxyl, nitro, cyano, trifluoromethyl, trifluoromethoxy, C₁₋₆ alkyl (as defined above), C₂₋₆ alkenyl (as defined above), C₂₋₆ alkynyl (as defined above), C₁₋₆ alkoxy, C₁₋₆ alkylthio, C₁₋₆ alkylsulfinyl, C₃₋₁₀ cycloalkyl (as defined above), C₃₋₁₀ cycloalkyl C₁₋₆ alkyl (as defined above), aryl (as defined above), aryloxy, aryl C₁₋₆ alkyl (as defined above), aryl C₂₋₆ alkenyl (as defined above), aryl C₂₋₆ alkynyl, heteroaryl (as defined above), heteroaryloxy, heteroaryl C₁₋₆ alkyl (as defined above), heterocycle (as defined above), oxo, -COORₐ, -CH₂COORₐ, -OCH₂COORₐ, -CONR_{b}R_{c}, -CH₂CONR_{b}R_{c}, -OCH₂CONR_{b}R_{c}, -COO(CH₂)₂NRₑR_{f}, -CONR_{d}SO₂T₁, -NRₑR_{f}, -NR_{g}CHO, -NR_{g}COT₂, -NR_{g}COOT₂, -NR_{g}CONRᵢRⱼ, -NRₕSO₂T₃, -NHC (=NH) NH₂, -COT₂, -SO₂T₃, methylenedioxy, and ethyleneoxy.

The above substituents may further have 1 to 3 substituents selected from the substituent group B at substitutable positions. When two or more substituents are present, they may be the same or different.

Substituent group B: a halogen atom (as defined above), hydroxyl, nitro, cyano, trifluoromethyl, trifluoromethoxy, C₁₋₆ alkyl (as defined above), C₂₋₆ alkenyl (as defined above), C₂₋₆ alkynyl (as defined above), C₁₋₆ alkoxy, C₁₋₆ alkylthio, C₁₋₆ alkylsulfinyl, C₃₋₁₀ cycloalkyl (as defined above), C₃₋₁₀ cycloalkyl C₁₋₆ alkyl (as defined above), aryl (as defined above), aryloxy, aryl C₁₋₆ alkyl (as defined above), aryl C₂₋₆ alkenyl (as defined above), aryl C₂₋₆ alkynyl, heteroaryl (as defined above), heteroaryloxy, heteroaryl C₁₋₆ alkyl (as defined above), heterocycle (as defined above), oxo, -COORₐ, -CH₂COORₐ, -OCH₂COORₐ, -CONR_{b}R_{c}, -CH₂CONR_{b}R_{c}, -OCH₂CONR_{b}R_{c}, -COO(CH₂)₂NRₑR_{f}, -CONR_{d}SO₂T₁, -NRₑR_{f}, -NR_{g}CHO, -NR_{g}COT₂, -NR_{g}COOT₂, -NR_{g}CONRᵢRⱼ, -NRₕSO₂T₃, -NHC (=NH) NH₂, -COT₂, -SO₂T₃, methylenedioxy, and ethyleneoxy.

The "C₁₋₆ alkyl" moiety of the C₁₋₆ alkoxy has the same meaning as the above "C₁₋₆ alkyl", and examples of the C₁₋₆ alkoxy include methoxy, ethoxy, propoxy, isopropoxy, butoxy, pentyloxy, hexyloxy, etc.

The "C₁₋₆ alkyl" moiety of the C₁₋₆ alkylthio has the same meaning as the above "C₁₋₆ alkyl", and examples of the alkylthio include methylthio, ethylthio, propylthio, isopropylthio, butylthio, pentylthio, hexylthio, etc.

The "C₁₋₆ alkyl" moiety of the C₁₋₆ alkylsulfinyl has the same meaning as the above "C₁₋₆ alkyl", and examples of the alkylsulfinyl include methylsulfinyl, ethylsulfinyl, propylsulfinyl, isopropylsulfinyl, butylsulfinyl, pentylsulfinyl, hexylsulfinyl, etc.

The aryl moiety of the aryloxy has the same meaning as the above "aryl", and examples of the aryloxy include phenoxy, 1-naphthoxy, 2-naphthoxy, etc.

The aryl C₂₋₆ alkynyl means the above "C₂₋₆ alkynyl" substituted with the above "aryl". The "C₂₋₆ alkynyl" is preferably an alkynyl having 2 to 4 carbon atoms, and examples thereof include phenylethynyl etc.

The "heteroaryl" moiety of the heteroaryloxy has the same meaning as the above "heteroaryl", and examples of the heteroaryloxy include 2-pyridyloxy, 2-benzothiazolyloxy, etc.

Rₐ to Rⱼ each denote a hydrogen atom, C₁₋₆ alkyl (as defined above), aryl (as defined above), aryl C₁₋₆ alkyl (as defined above), heteroaryl (as defined above), or heteroaryl C₁₋₆ alkyl (as defined above), and these groups may further have 1 to 3 substituents selected from the substituent group A at substitutable positions.

R_{b} and R_{c}, Rₑ and R_{f}, and Rᵢ and Rⱼ in -NR_{b}R_{c}, -NRₑR_{f}, and -NRᵢRⱼ may form a cyclic amino (as defined above) together with the nitrogen atom to which they are bonded, and the cyclic amino may further have 1 to 3 substituents selected from the substituent group A at substitutable positions. The cyclic amino that -NRₑR_{f} forms encompasses cyclic amino containing oxo (for example, 2-pyrrolidinon-1-yl, 1-oxoisoindolin-2-yl, succinimide, oxazolidin-2-on-3-yl, 2-benzoxazolinon-3-yl, phthalimide, 4-quinazolinon-3-yl, etc.).

T₁ to T₃ each denote C₁₋₆ alkyl (as defined above), C₂₋₆ alkenyl (as defined above), C₂₋₆ alkynyl (as defined above), C₃₋₁₀ cycloalkyl (as defined above), C₃₋₁₀ cycloalkyl C₁₋₆ alkyl (as defined above), aryl (as defined above), aryl C₁₋₆ alkyl (as defined above), heteroaryl (as defined above), heteroaryl C₁₋₆ alkyl (as defined above), cyclic amino (as defined above), or heterocycle (as defined above), and these groups may further have 1 to 3 substituents selected from the substituent group A at substitutable positions. Examples of the aryl or heteroaryl having 1 to 3 substituents selected from the substituent group A include 2-aminophenyl, 2-amino-5-fluorophenyl, 2-amino-6-fluorophenyl, 2-fluorophenyl, 4-methoxypheny, 5-chloro-2-pyridyl, etc.

Particularly preferred AT₂ receptor agonists as an active ingredient of the present invention are the following example compounds 1 to 588 as disclosed in WO 2008/156142.

In the following chemical formulae, MeO denotes a methoxy group, ph denotes a phenyl group, and TFA denotes trifluoroacetic acid.

| Table 1 | | 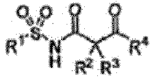 | ↑ :Same substituent as above | |
|---|---|---|---|---|
| Example | R¹ | R² | R³ | R⁴ |
| 1 | 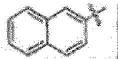 | 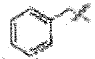 | H | 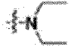 |
| 2 | ↑ | ↑ | ↑ | 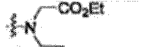 |
| 3 | ↑ | ↑ | ↑ | 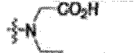 |
| 4 | ↑ | ↑ | ↑ | 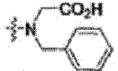 |
| 5 | ↑ | ↑ | ↑ | 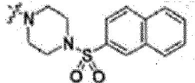 |
| 6 | ↑ | 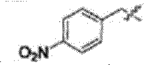 | ↑ | 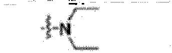 |
| 7 | ↑ | 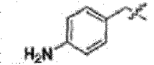 | ↑ | ↑ |
| 8 | ↑ | 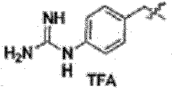 | ↑ | ↑ |

| Table 2 | 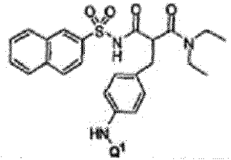 | | |
|---|---|---|---|
| Example | Q¹ | Example | Q¹ |
| 9 | PhCO | 17 | 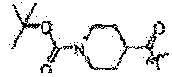 |
| 10 | Ac | 18 | 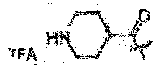 |
| 11 | 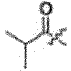 | 19 | 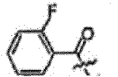 |
| 12 | 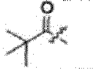 | 20 | 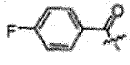 |
| 13 | 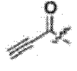 . | 21 | 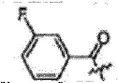 |
| 14 | 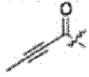 | 22 | 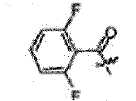 |
| 15 | 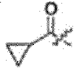 | 23 | 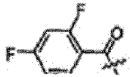 |
| 16 | 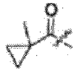 | 24 | 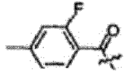 |

| Table 2 (-continued) | | | |
|---|---|---|---|
| 25 | 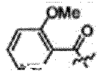 | 36 | 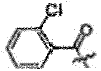 |
| 26 | 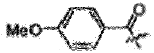 | 37 | 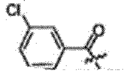 |
| 27 | 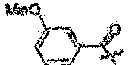 | 38 | 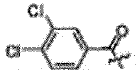 |
| 28 | 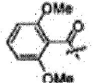 | 39 | 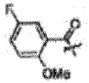 |
| 29 | 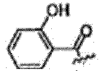 | 40 | 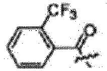 |
| 30 | 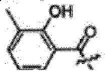 | 41 | 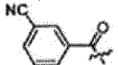 |
| 31 | 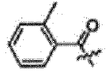 | 42 | 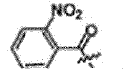 |
| 32 | 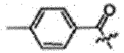 | 43 | 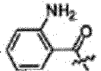 |
| 33 | 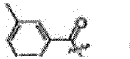 | 44 | 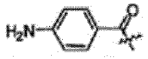 |
| 34 | 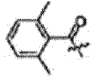 | 46 | 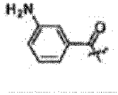 |
| 35 | 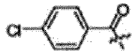 | 46 | 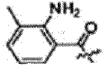 |
| 47 | 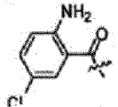 | 56 | 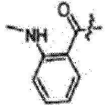 |
| 48 | 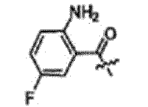 | 57 | 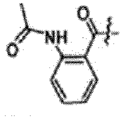 |
| 49 | 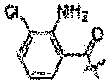 | 58 | 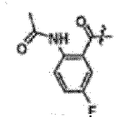 |
| 50 | 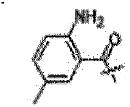 | 59 | 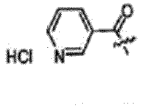 |
| 51 | 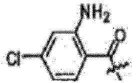 | 60 | 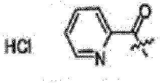 |
| 52 | 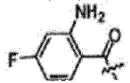 | 61 | 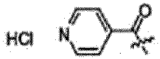 |
| 53 | 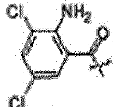 | 62 | 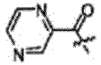 |
| 54 | 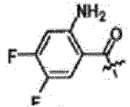 | 63 | 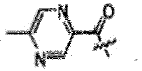 |
| 55 | 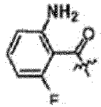 | 64 | 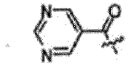 |
| 65 | 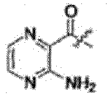 | 74 | 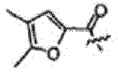 |
| 66 | 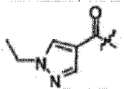 | 75 | 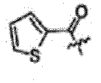 |
| 67 | 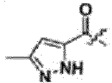 | 76 | 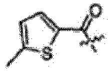 |
| 68 | 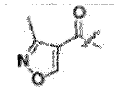 | 77 | 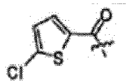 |
| 69 | 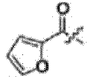 | 78 | 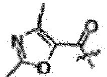 |
| 70 | 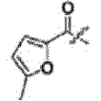 | 79 | 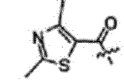 |
| 71 | 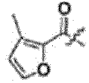 | 80 | 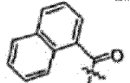 |
| 72 | 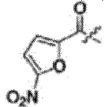 | 81 | 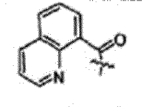 |
| 73 | 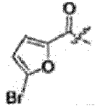 | 82 | 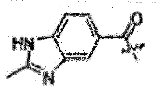 |
| 82 | 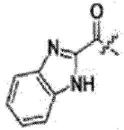 | 87 | PhOCO |
| 84 | 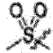 | 88 | PhNHCO |
| 85 | 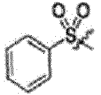 | 89 | 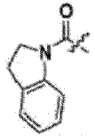 |
| 86 | 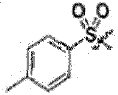 | 90 | 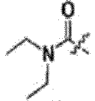 |

| Table 3 | 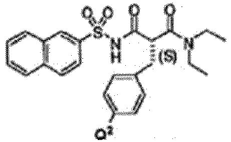 | | |
|---|---|---|---|
| Example | Q² | Example | Q² |
| 91 | NO₂ | 100 | 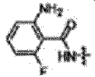 |
| 92 | PhCONH | 101 | 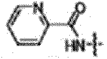 |
| 93 | 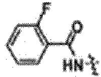 | 102 | NH₂ |
| 94 | 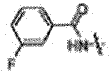 | 103 | 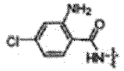 |
| 96 | 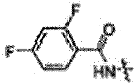 | 104 | 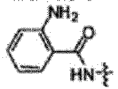 |
| 96 | 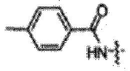 | 105 | 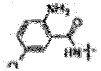 |
| 97 | 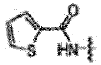 | 106 | 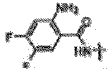 |
| 98 | 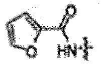 | 107 | 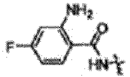 |
| 99 | 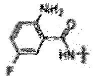 | 108 | 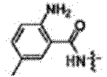 |

| Table 4 | | 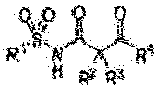 | ↑ :Same substituent as above | |
|---|---|---|---|---|
| Example | R¹ | R² | R³ | R⁴ |
| 109 | 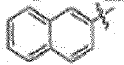 | 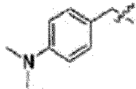 | H | 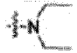 |
| 110 | ↑ | 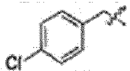 | ↑ | ↑ |
| 111 | ↑ | 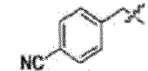 | ↑ | ↑ |
| 112 | ↑ | 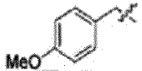 | ↑ | ↑ |
| 113 | ↑ | 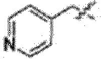 | ↑ | ↑ |
| 114 | ↑ | 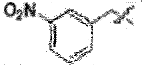 | ↑ | ↑ |
| 115 | ↑ | 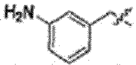 | ↑ | ↑ |
| 118 | ↑ | 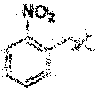 | ↑ | ↑ |
| 117 | ↑ | 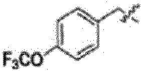 | ↑ | ↑ |
| 118 | ↑ | 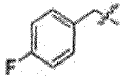 | ↑ | ↑ |
| 119 | ↑ | 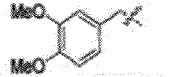 | ↑ | ↑ |
| 120 | ↑ | 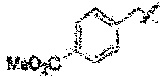 | ↑ | ↑ |
| 121 | ↑ | 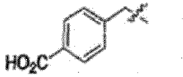 | ↑ | ↑ |
| 122 | ↑ | 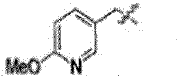 | ↑ | ↑ |
| 123 | ↑ | 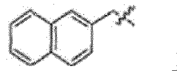 | ↑ | ↑ |
| 124 | ↑ | 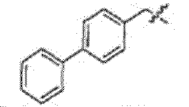 | ↑ | ↑ |
| 125 | t | 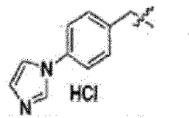 | ↑ | ↑ |
| 128 | ↑ | 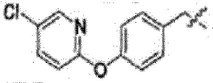 | ↑ | ↑ |
| 127 | t | 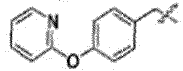 | ↑ | ↑ |
| 128 | ↑ | 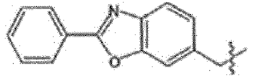 | ↑ | ↑ |
| 129 | t | 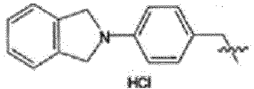 | ↑ | ↑ |
| 130 | ↑ | 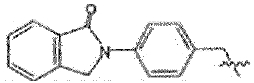 | ↑ | ↑ |
| 131 | ↑ | 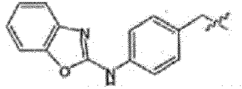 | ↑ | ↑ |
| 132 | ↑ | 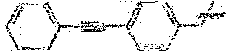 | ↑ | ↑ |
| 133 | ↑ | 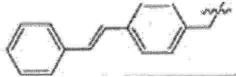 | ↑ | ↑ |
| 134 | ↑ | 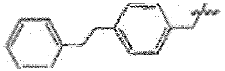 | ↑ | ↑ |
| 135 | ↑ | 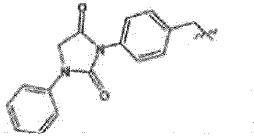 | ↑ | ↑ |
| 136 | ↑ | 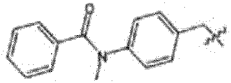 | ↑ | ↑ |

| Table 4 (-continued) | | | | |
|---|---|---|---|---|
| 137 | ↑ | 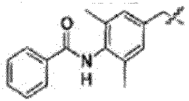 | ↑ | ↑ |
| 138 | ↑ | 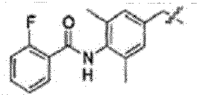 | ↑ | ↑ |
| 139 | ↑ | 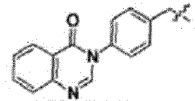 | ↑ | ↑ |
| 140 | ↑ | 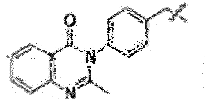 | ↑ | ↑ |
| 141 | ↑ | 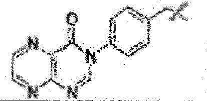 | ↑ | ↑ |
| 142 | ↑ | 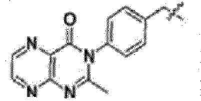 | ↑ | ↑ |
| 143 | ↑ | 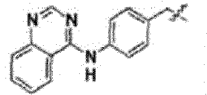 | ↑ | ↑ |
| 144 | ↑ | 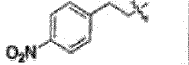 | ↑ | ↑ |
| 145 | ↑ | 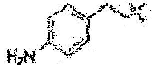 | ↑ | ↑ |
| 148 | ↑ | 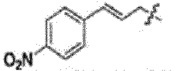 | ↑ | ↑ |
| 147 | ↑ | 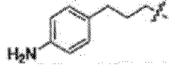 | ↑ | ↑ |
| 148 | ↑ | 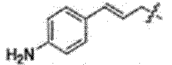 | ↑ | ↑ |
| 149 | ↑ | 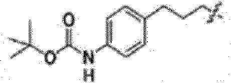 | ↑ | ↑ |
| 150 | ↑ | 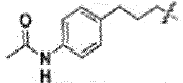 | ↑ | ↑ |
| 151 | ↑ | 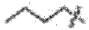 | ↑ | ↑ |
| 152 | ↑ | 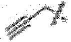 | ↑ | ↑ |
| 153 | ↑ | 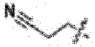 | ↑ | ↑ |
| 154 | ↑ | 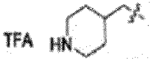 | ↑ | ↑ |
| 158 | ↑ | 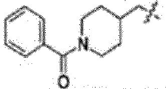 | ↑ | ↑ |
| 156 | ↑ | 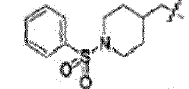 | ↑ | ↑ |
| 157 | ↑ | 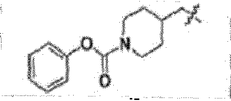 | ↑ | ↑ |
| 158 | ↑ | 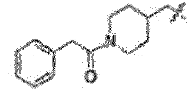 | ↑ | ↑ |
| 159 | ↑ | 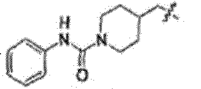 | ↑ | ↑ |
| 160 | ↑ | 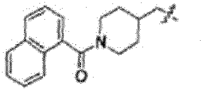 | ↑ | ↑ |
| 161 | ↑ | 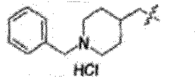 | ↑ | ↑ |
| 162 | ↑ | 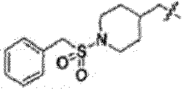 | ↑ | ↑ |
| 163 | ↑ | 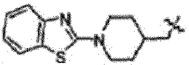 | ↑ | ↑ |
| 164 | ↑ | 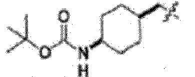 | ↑ | ↑ |
| 165 | ↑ | 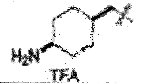 | ↑ | ↑ |
| 166 | ↑ | 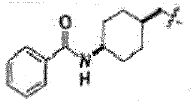 | ↑ | ↑ |
| 167 | ↑ | 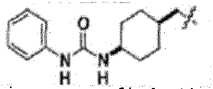 | ↑ | ↑ |
| 168 | ↑ | 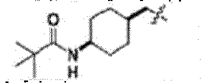 | ↑ | ↑ |
| 169 | ↑ | 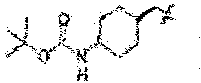 | ↑ | ↑ |
| 170 | ↑ | 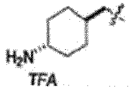 | ↑ | ↑ |
| 171 | ↑ | 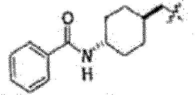 | ↑ | t |
| 172 | ↑ | 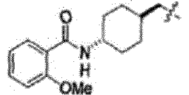 | ↑ | ↑ |
| 173 | ↑ | 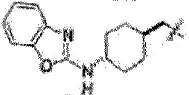 | ↑ | ↑ |
| 174 | ↑ | 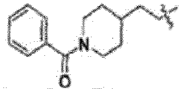 | ↑ | ↑ |
| 175 | ↑ | 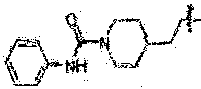 | ↑ | ↑ |
| 176 | ↑ | 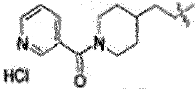 | ↑ | ↑ |
| 177 | 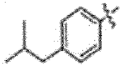 | 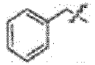 | ↑ | ↑ |
| 178 | 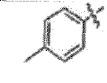 | 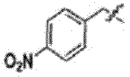 | ↑ | t |
| 179 | ↑ | 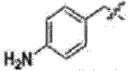 | ↑ | ↑ |
| 180 | 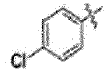 | 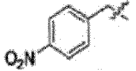 | ↑ | ↑ |
| 181 | ↑ | 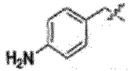 | ↑ | ↑ |
| 182 | 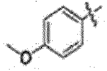 | 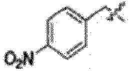 | ↑ | ↑ |
| 183 | ↑ | 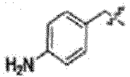 | ↑ | ↑ |
| 184 | 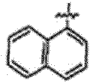 | 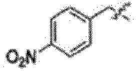 | ↑ | ↑ |
| 185 | ↑ | 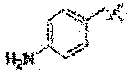 | ↑ | ↑ |
| 186 | 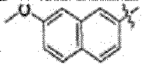 | 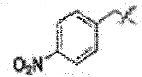 | ↑ | ↑ |
| 187 | ↑ | 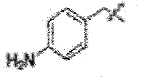 | ↑ | ↑ |
| 188 | 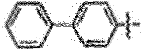 | 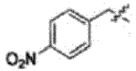 | ↑ | ↑ |
| 189 | ↑ | 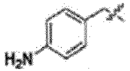 | ↑ | ↑ |
| 190 | 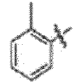 | 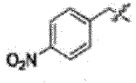 | ↑ | ↑ |
| 191 | ↑ | 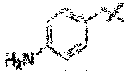 | ↑ | ↑ |
| 192 | 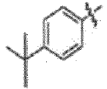 | 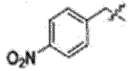 | ↑ | ↑ |
| 193 | ↑ | 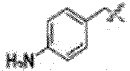 | ↑ | ↑ |
| 194 | 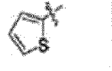 | 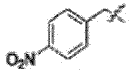 | ↑ | ↑ |
| 195 | ↑ | 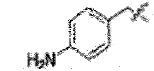 | ↑ | ↑ |
| 196 | 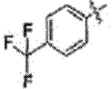 | 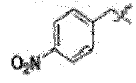 | ↑ | ↑ |
| 197 | ↑ | 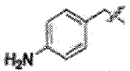 | ↑ | ↑ |
| 198 | 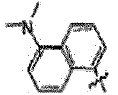 | 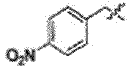 | ↑ | ↑ |
| 199 | ↑ | 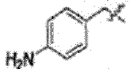 | ↑ | ↑ |
| 200 | 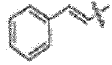 | 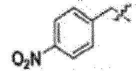 | ↑ | ↑ |
| 201 | ↑ | 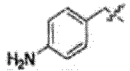 | ↑ | ↑ |
| 202 | ↑ | 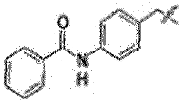 | ↑ | ↑ |
| 203 | 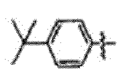 | ↑ | ↑ | ↑ |
| 204 | 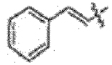 | 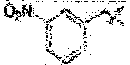 | ↑ | ↑ |
| 205 | ↑ | 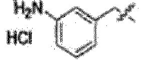 | ↑ | ↑ |
| 206 | ↑ | 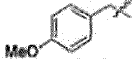 | ↑ | ↑ |
| 207 | ↑ | 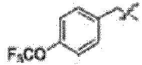 | ↑ | ↑ |
| 208 | ↑ | 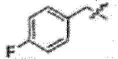 | ↑ | ↑ |
| 209 | ↑ | 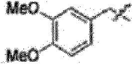 | ↑ | ↑ |
| 210 | ↑ | 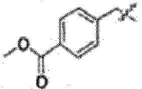 | ↑ | ↑ |
| 211 | ↑ | 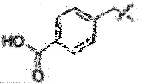 | ↑ | ↑ |
| 212 | ↑ | 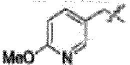 | ↑ | ↑ |
| 218 | ↑ | 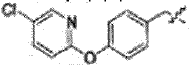 | ↑ | ↑ |
| 214 | ↑ | 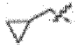 | ↑ | ↑ |
| 215 | ↑ | 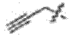 | ↑ | ↑ |
| 216 | ↑ | 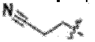 | ↑ | ↑ |
| 217 | ↑ | 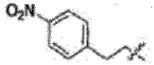 | ↑ | ↑ |
| 218 | ↑ | 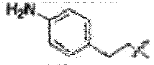 | ↑ | ↑ |
| 219 | 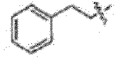 | 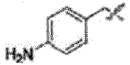 | ↑ | ↑ |
| 220 | ↑ | 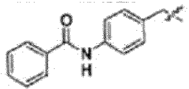 | ↑ | ↑ |
| 221 | ↑ | 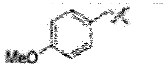 | ↑ | ↑ |
| 222 | ↑ | 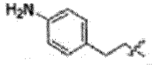 | ↑ | ↑ |
| 223 | 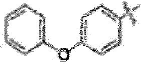 | 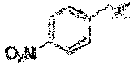 | ↑ | ↑ |
| 224 | ↑ | 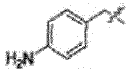 | ↑ | ↑ |
| 225 | 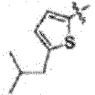 | 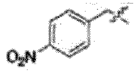 | ↑ | ↑ |
| 228 | ↑ | 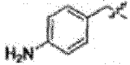 | ↑ | ↑ |
| 227 | 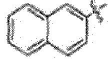 | 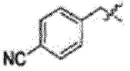 | ↑ | 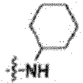 |
| 228 | ↑ | ↑ | ↑ | 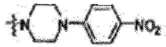 |
| 229 | ↑ | ↑ | ↑ | 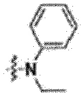 |
| 230 | ↑ | ↑ | ↑ | 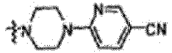 |
| 231 | ↑ | ↑ | ↑ | 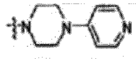 |
| 232 | ↑ | 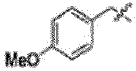 | ↑ | 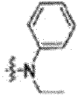 |
| 233 | ↑ | ↑ | ↑ | 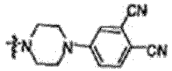 |
| 234 | ↑ | ↑ | ↑ | 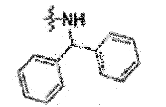 |
| 235 | ↑ | 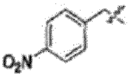 | ↑ | 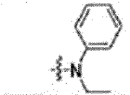 |
| 236 | t | 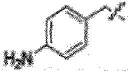 | ↑ | ↑ |
| 237 | ↑ | 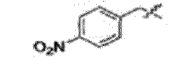 | | 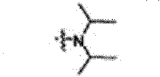 |
| 238 | ↑ | 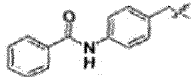 | ↑ | ↑ |
| 239 | ↑ | ↑ | | 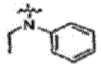 |
| 240 | ↑ | ↑ | ↑ | 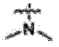 |
| 241 | ↑ | ↑ | ↑ | 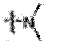 |
| 242 | ↑ | ↑ | ↑ | 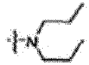 |
| 243 | ↑ | ↑ | ↑ | 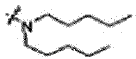 |
| 244 | ↑ | ↑ | ↑ | 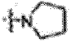 |
| 245 | ↑ | ↑ | ↑ | 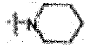 |
| 246 | ↑ | ↑ | ↑ | 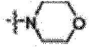 |
| 247 | ↑ | ↑ | ↑ | 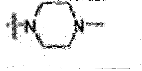 |
| 248 | ↑ | ↑ | ↑ | 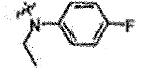 |
| 249 | ↑ | ↑ | ↑ | 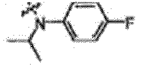 |
| 250 | ↑ | ↑ | ↑ | 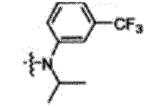 |
| 251 | ↑ | ↑ | ↑ | 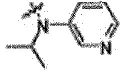 |
| 252 | ↑ | 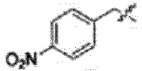 | ↑ | 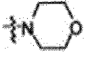 |
| 253 | ↑ | 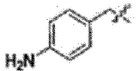 | ↑ | 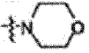 |
| 254 | ↑ | 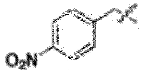 | ↑ | 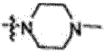 |
| 253 | ↑ | 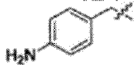 | ↑ | 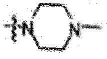 |
| 256 | ↑ | 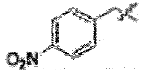 | ↑ | 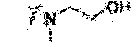 |
| 257 | ↑ | 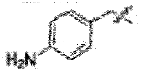 | ↑ | 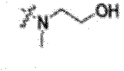 |
| 258 | ↑ | 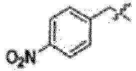 | ↑ | 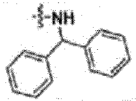 |
| 259 | ↑ | 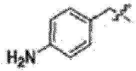 | ↑ | 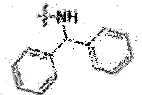 |
| 260 | ↑ | 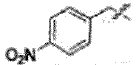 | ↑ | 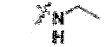 |
| 261 | ↑ | 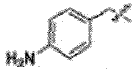 | ↑ | 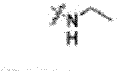 |
| 262 | ↑ | 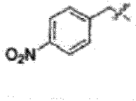 | ↑ | 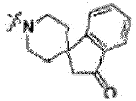 |
| 263 | ↑ | 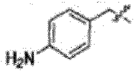 | ↑ | 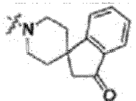 |
| 264 | ↑ | 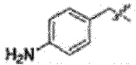 | ↑ | 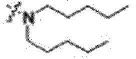 |
| 265 | 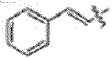 | 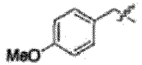 | ↑ | 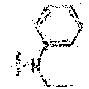 |
| 266 | 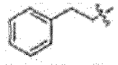 | 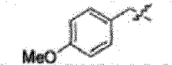 | ↑ | ↑ |
| 267 | 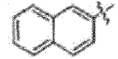 | 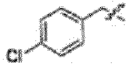 | ↑ | ↑ |
| 268 | ↑ | 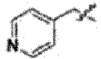 | ↑ | ↑ |
| 269 | ↑ | 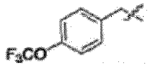 | ↑ | ↑ |
| 270 | 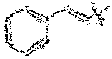 | 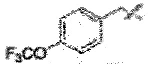 | ↑ | ↑ |
| 271 | 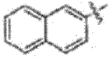 | 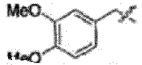 | ↑ | ↑ |
| 272 | 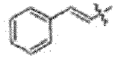 | 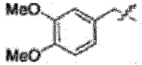 | ↑ | ↑ |
| 273 | 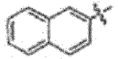 | 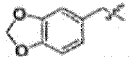 | ↑ | ↑ |
| 274 | 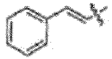 | 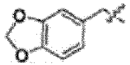 | ↑ | ↑ |
| 275 | 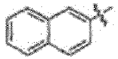 | 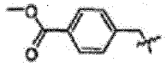 | ↑ | ↑ |
| 276 | ↑ | 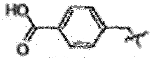 | ↑ | ↑ |
| 277 | 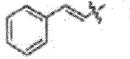 | 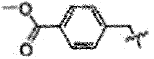 | ↑ | ↑ |
| 278 | ↑ | 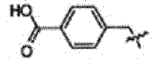 | ↑ | ↑ |
| 279 | 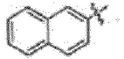 | 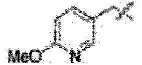 | ↑ | ↑ |
| 280 | 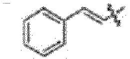 | 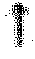 | ↑ | ↑ |
| 281 | ↑ | 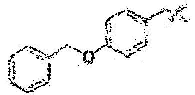 | ↑ | ↑ |
| 282 | ↑ | 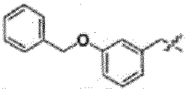 | ↑ | ↑ |
| 283 | ↑ | 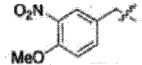 | ↑ | ↑ |
| 284 | 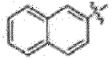 | 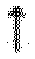 | ↑ | ↑ |
| 285 | ↑ | 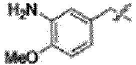 | ↑ | ↑ |
| 286 | 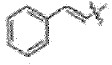 | 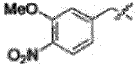 | ↑ | ↑ |
| 287 | 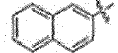 | ↑ | ↑ | ↑ |
| 288 | ↑ | 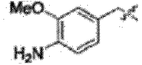 | ↑ | ↑ |
| 289 | 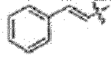 | 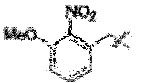 | ↑ | ↑ |
| 290 | ↑ | 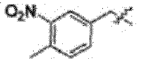 | ↑ | ↑ |
| 291 | ↑ | 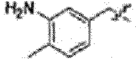 | ↑ | ↑ |
| 292 | ↑ | 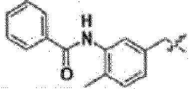 | ↑ | ↑ |
| 293 | 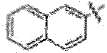 | 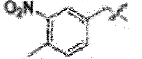 | ↑ | ↑ |
| 294 | ↑ | 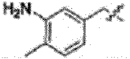 | ↑ | ↑ |
| 295 | ↑ | 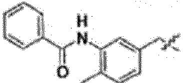 | ↑ | ↑ |
| 296 | ↑ | 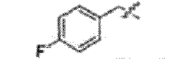 | ↑ | ↑ |
| 297 | 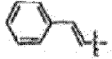 | ↑ | ↑ | ↑ |
| 298 | ↑ | 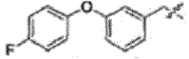 | ↑ | ↑ |
| 299 | ↑ | 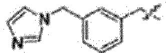 | ↑ | ↑ |
| 800 | ↑ | 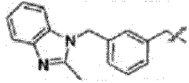 | ↑ | ↑ |
| 301 | ↑ | 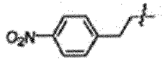 | ↑ | ↑ |
| 302 | ↑ | 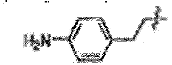 | ↑ | ↑ |
| 303 | 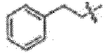 | 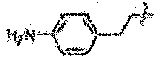 | ↑ | ↑ |
| 304 | 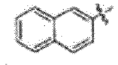 | 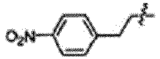 | ↑ | ↑ |
| 305 | ↑ | 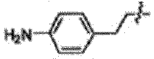 | ↑ | ↑ |
| 306 | 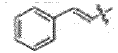 | 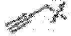 | ↑ | ↑ |
| 307 | 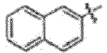 | ↑ | ↑ | ↑ |
| 308 | 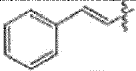 | 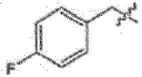 | ↑ | 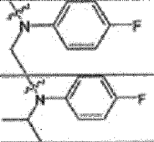 |
| 309 | ↑ | ↑ | ↑ | |
| 310 | ↑ | 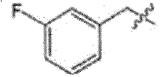 | ↑ | ↑ |
| 311 | 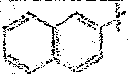 | ↑ | ↑ | ↑ |
| 312 | 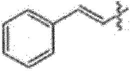 | 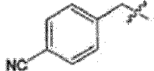 | ↑ | ↑ |
| 313 | 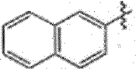 | ↑ | ↑ | ↑ |
| 314 | 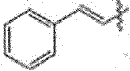 | 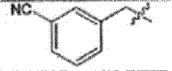 | ↑ | ↑ |
| 315 | 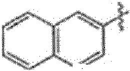 | ↑ | ↑ | ↑ |
| 316 | ↑ | 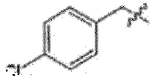 | ↑ | ↑ |
| 317 | ↑ | ↑ | ↑ | 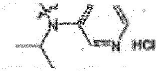 |
| 318 | 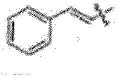 | 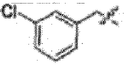 | ↑ | 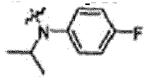 |
| 319 | 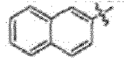 | ↑ | ↑ | ↑ |
| 320 | 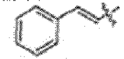 | 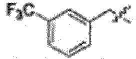 | ↑ | ↑ |
| 321 | 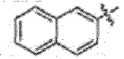 | ↑ | ↑ | ↑ |
| 322 | 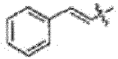 | 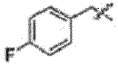 | ↑ | 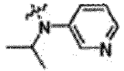 |
| 323 | ↑ | ↑ | ↑ | 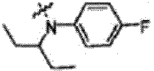 |
| 324 | ↑ | ↑ | ↑ | 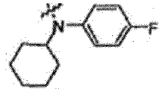 |
| 325 | ↑ | ↑ | ↑ | 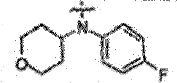 |
| 326 | ↑ | ↑ | ↑ | 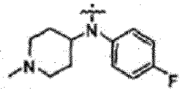 |
| 327 | ↑ | ↑ | ↑ | 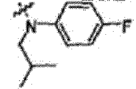 |
| 328 | ↑ | ↑ | ↑ | 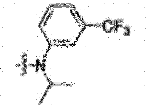 |
| 329 | ↑ | ↑ | ↑ | 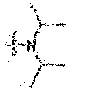 |
| 330 | ↑ | ↑ | ↑ | 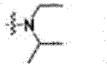 |
| 331 | 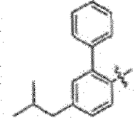 | 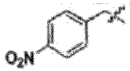 | ↑ | 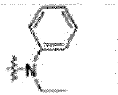 |
| 332 | ↑ | 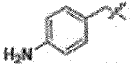 | ↑ | ↑ |
| 333 | 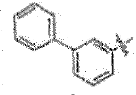 | 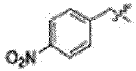 | ↑ | ↑ |
| 334 | ↑ | 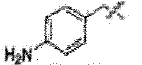 | ↑ | ↑ |
| 335 | 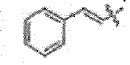 | 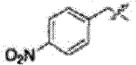 | ↑ | ↑ |
| 336 | ↑ | 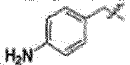 | ↑ | ↑ |
| 337 | ↑ | 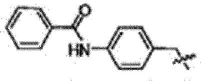 | ↑ | ↑ |
| 338 | 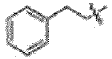 | 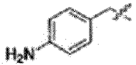 | ↑ | ↑ |
| 339 | 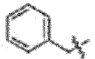 | 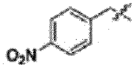 | ↑ | ↑ |
| 340 | ↑ | 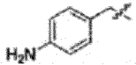 | ↑ | ↑ |
| 341 | 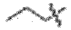 | 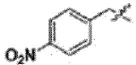 | ↑ | ↑ |
| 342 | ↑ | 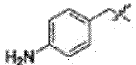 | ↑ | ↑ |
| 343 | 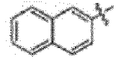 | 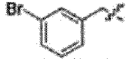 | ↑ | ↑ |
| 344 | 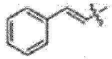 | ↑ | ↑ | ↑ |
| 345 | ↑ | 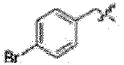 | ↑ | ↑ |
| 346 | 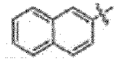 | ↑ | ↑ | ↑ |
| 347 | 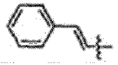 | 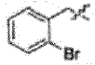 | ↑ | ↑ |
| 348 | 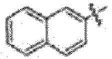 | ↑ | ↑ | ↑ |
| 349 | 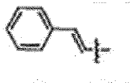 | 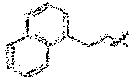 | ↑ | ↑ |
| 350 | 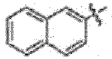 | ↑ | ↑ | ↑ |
| 351 | 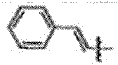 | 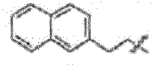 | ↑ | ↑ |
| 352 | 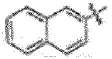 | ↑ | ↑ | ↑ |
| 353 | 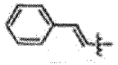 | 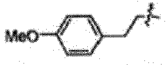 | ↑ | ↑ |
| 354 | 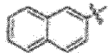 | ↑ | ↑ | ↑ |
| 355 | 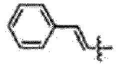 | 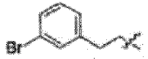 | ↑ | ↑ |
| 356 | 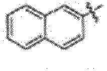 | ↑ | ↑ | ↑ |
| 357 | 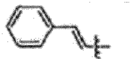 | 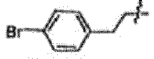 | ↑ | ↑ |
| 358 | 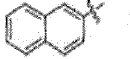 | ↑ | ↑ | ↑ |
| 359 | 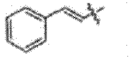 | 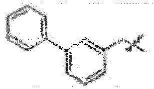 | ↑ | ↑ |
| 360 | 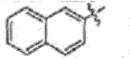 | ↑ | ↑ | ↑ |
| 361 | 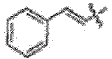 | 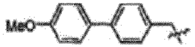 | ↑ | ↑ |
| 362 | 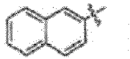 | ↑ | ↑ | ↑ |
| 363 | 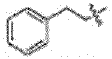 | ↑ | ↑ | ↑ |
| 364 | 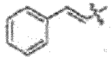 | 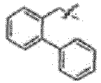 | ↑ | ↑ |
| 365 | 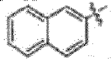 | ↑ | ↑ | ↑ |
| 366 | 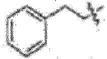 | ↑ | ↑ | ↑ |
| 367 | 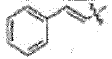 | 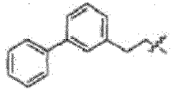 | ↑ | ↑ |
| 368 | ↑ | 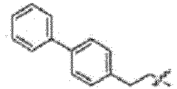 | ↑ | ↑ |
| 869 | 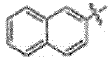 | ↑ | ↑ | ↑ |
| 370 | ↑ | 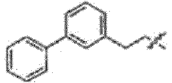 | ↑ | ↑ |
| 371 | 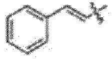 | 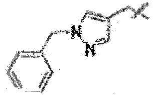 | ↑ | ↑ |
| 372 | ↑ | 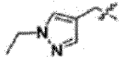 | ↑ | ↑ |
| 373 | ↑ | 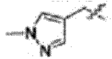 | ↑ | ↑ |
| 374 | ↑ | 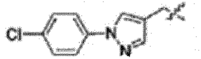 | ↑ | ↑ |
| 375 | ↑ | 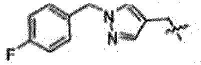 | ↑ | ↑ |
| 376 | ↑ | 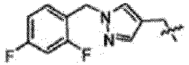 | ↑ | ↑ |
| 377 | ↑ | 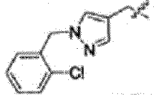 | ↑ | ↑ |
| 378 | ↑ | 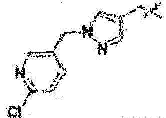 | ↑ | ↑ |
| 379 | ↑ | 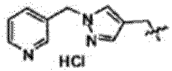 | ↑ | ↑ |
| 380 | ↑ | 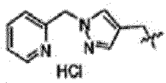 | ↑ | ↑ |
| 381 | ↑ | 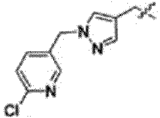 | ↑ | 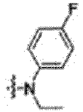 |
| 382 | ↑ | 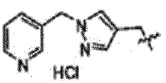 | ↑ | ↑ |
| 383 | ↑ | 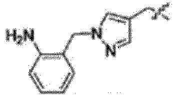 | ↑ | 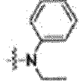 |
| 884 | ↑ | 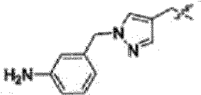 | ↑ | ↑ |
| 385 | ↑ | 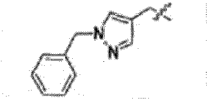 | ↑ | 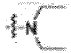 |
| 386 | ↑ | 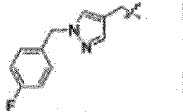 | ↑ | ↑ |
| 387 | ↑ | 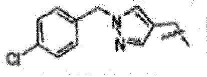 | ↑ | ↑ |
| 388 | ↑ | 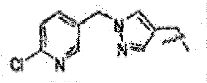 | ↑ | ↑ |
| 389 | 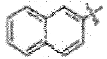 | 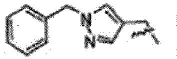 | ↑ | ↑ |
| 390 | ↑ | 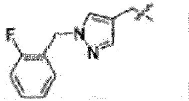 | ↑ | ↑ |
| 391 | ↑ | 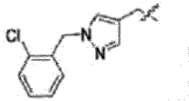 | ↑ | ↑ |
| 392 | ↑ | 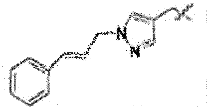 | ↑ | ↑ |
| 393 | ↑ | 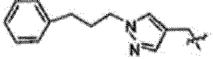 | ↑ | ↑ |
| 394 | 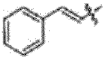 | 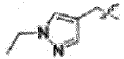 | ↑ | 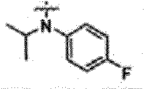 |
| 896 | ↑ | 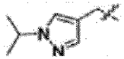 | ↑ | ↑ |
| 396 | ↑ | ↑ | ↑ | 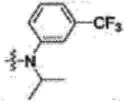 |
| 397 | 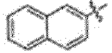 | ↑ | ↑ | 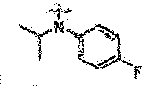 |
| 398 | 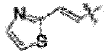 | 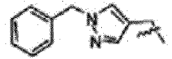 | ↑ | 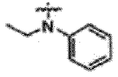 |
| 399 | 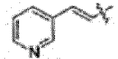 | ↑ | ↑ | ↑ |
| 400 | 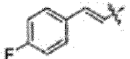 | ↑ | ↑ | ↑ |
| 401 | 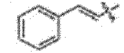 | 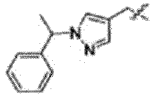 | ↑ | ↑ |
| 402 | | 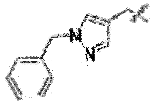 | ↑ | ↑ |
| 403 | 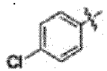 | ↑ | ↑ | ↑ |
| 404 | 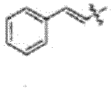 | 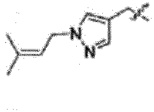 | ↑ | 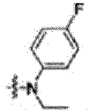 |
| 405 | 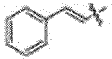 | 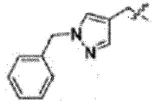 | ↑ | 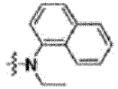 |
| 406 | ↑ | ↑ | ↑ | 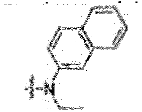 |
| 407 | ↑ | ↑ | ↑ | 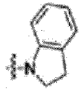 |
| 408 | ↑ | ↑ | ↑ | 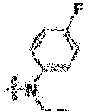 |
| 409 | ↑ | ↑ | ↑ | 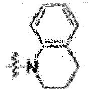 |
| 410 | ↑ | ↑ | ↑ | 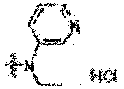 |
| 411 | ↑ | ↑ | ↑ | 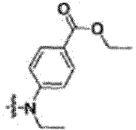 |
| 412 | ↑ | ↑ | ↑ | 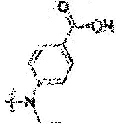 |
| 413 | ↑ | ↑ | ↑ | 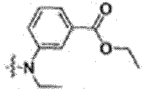 |
| 414 | ↑ | ↑ | ↑ | 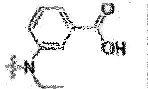 |
| 415 | ↑ | ↑ | ↑ | 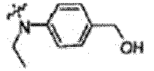 |
| 416 | ↑ | ↑ | ↑ | 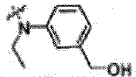 |
| 417 | ↑ | ↑ | ↑ | 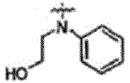 |
| 418 | ↑ | ↑ | ↑ | 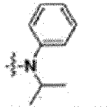 |
| 419 | ↑ | ↑ | ↑ | 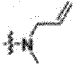 |
| 420 | 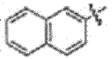 | ↑ | ↑ | 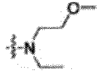 |
| 421 | ↑ | ↑ | ↑ | 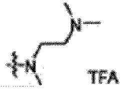 |
| 422 | ↑ | ↑ | ↑ | 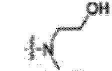 |
| 423 | ↑ | ↑ | ↑ | 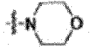 |
| 424 | ↑ | ↑ | ↑ | 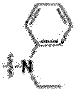 |
| 425 | 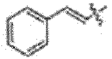 | 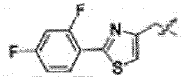 | ↑ | ↑ |
| 426 | ↑ | 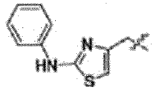 | ↑ | ↑ |
| 427 | ↑ | 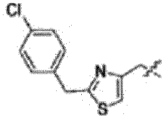 | ↑ | 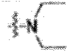 |
| 428 | ↑ | 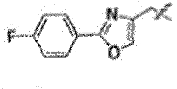 | ↑ | 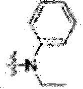 |
| 429 | ↑ | 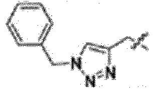 | ↑ | ↑ |
| 430 | ↑ | ↑ | ↑ | 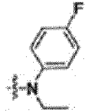 |
| 431 | ↑ | 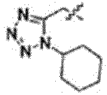 | ↑ | 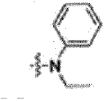 |
| 432 | ↑ | 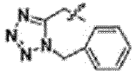 | ↑ | ↑ |
| 433 | ↑ | 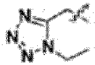 | ↑ | 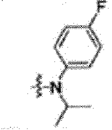 |
| 434 | ↑ | 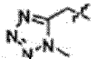 | ↑ | ↑ |
| 435 | ↑ | 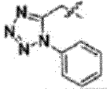 | ↑ | ↑ |
| 436 | 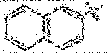 | ↑ | ↑ | 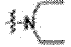 |
| 437 | ↑ | 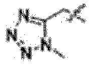 | ↑ | 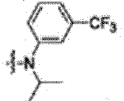 |
| 438 | 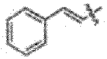 | 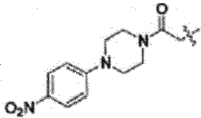 | ↑ | 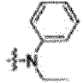 |
| 439 | ↑ | 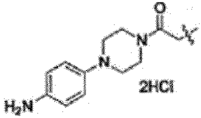 | ↑ | ↑ |
| 440 | ↑ | 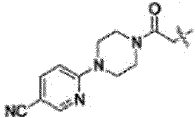 | ↑ | ↑ |
| 441 | ↑ | 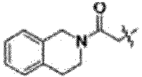 | ↑ | ↑ |
| 442 | ↑ | 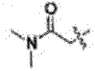 | ↑ | 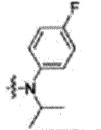 |
| 443 | ↑ | 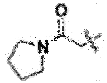 | ↑ | ↑ |
| 444 | 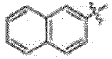 | 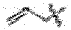 | ↑ | 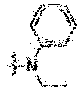 |
| 445 | 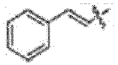 | ↑ | ↑ | 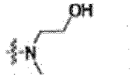 |
| 446 | ↑ | ↑ | ↑ | 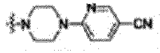 |
| 447 | ↑ | ↑ | ↑ | 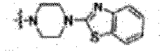 |
| 448 | ↑ | ↑ | ↑ | 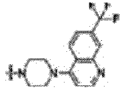 |
| 449 | ↑ | ↑ | ↑ | 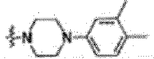 |
| 450 | ↑ | ↑ | ↑ | 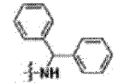 |
| 451 | ↑ | ↑ | ↑ | 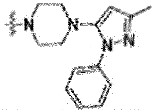 |
| 452 | ↑ | ↑ | ↑ | 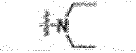 |
| 453 | ↑ | ↑ | ↑ | 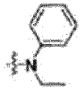 |
| 454 | ↑ | ↑ | ↑ | 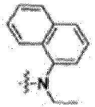 |
| 455 | 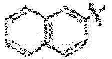 | ↑ | ↑ | ↑ |
| 456 | 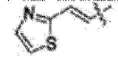 | ↑ | ↑ | 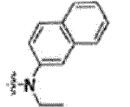 |
| 457 | ↑ | ↑ | ↑ | 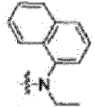 |
| 458 | 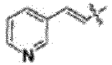 | ↑ | ↑ | 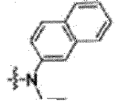 |
| 459 | 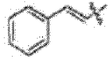 | ↑ | ↑ | 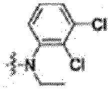 |
| 460 | ↑ | ↑ | ↑ | 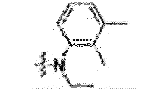 |
| 461 | ↑ | ↑ | ↑ | 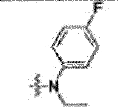 |
| 462 | ↑ | ↑ | ↑ | 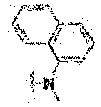 |
| 463 | ↑ | ↑ | ↑ | 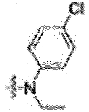 |
| 464 | ↑ | ↑ | ↑ | 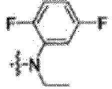 |
| 465 | ↑ | ↑ | ↑ | 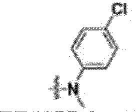 |
| 466 | ↑ | ↑ | ↑ | 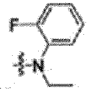 |
| 467 | ↑ | ↑ | ↑ | 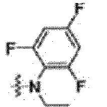 |
| 468 | ↑ | ↑ | ↑ | 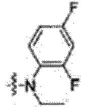 |
| 469 | ↑ | ↑ | ↑ | 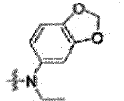 |
| 470 | ↑ | ↑ | ↑ | 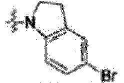 |
| 471 | ↑ | ↑ | ↑ | 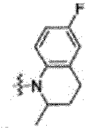 |
| 472 | ↑ | ↑ | ↑ | 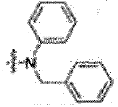 |
| 473 | ↑ | ↑ | ↑ | 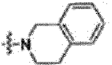 |
| 474 | ↑ | ↑ | ↑ | 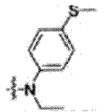 |
| 475 | ↑ | ↑ | ↑ | 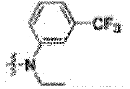 |
| 476 | ↑ | ↑ | ↑ | 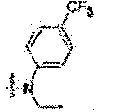 |
| 477 | ↑ | ↑ | ↑ | 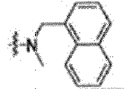 |
| 478 | ↑ | ↑ | ↑ | 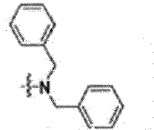 |
| 479 | ↑ | ↑ | ↑ | 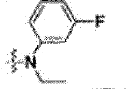 |
| 480 | ↑ | ↑ | ↑ | 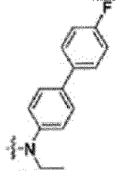 |
| 481 | ↑ | ↑ | ↑ | 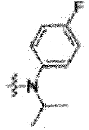 |
| 482 | ↑ | ↑ | ↑ | 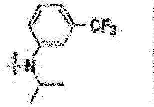 |
| 483 | ↑ | ↑ | ↑ | 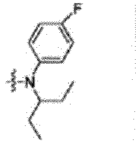 |
| 484 | ↑ | ↑ | ↑ | 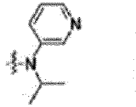 |
| 485 | ↑ | ↑ | ↑ | 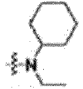 |
| 486 | ↑ | 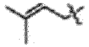 | ↑ | 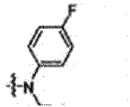 |
| 487 | ↑ | 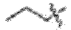 | ↑ | 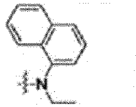 |
| 488 | ↑ | ↑ | ↑ | 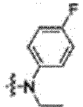 |
| 489 | ↑ | ↑ | ↑ | 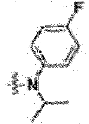 |
| 490 | ↑ | 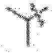 | ↑ | ↑ |
| 491 | ↑ | 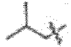 | ↑ | ↑ |
| 492 | 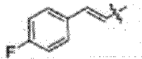 | ↑ | ↑ | ↑ |
| 493 | 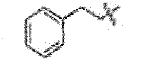 | ↑ | ↑ | ↑ |
| 494 | 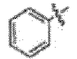 | ↑ | ↑ | ↑ |
| 495 | 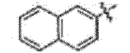 | ↑ | ↑ | ↑ |
| 496 | 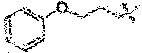 | ↑ | ↑ | ↑ |
| 497 | 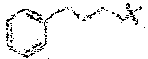 | ↑ | ↑ | ↑ |
| 498 | 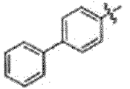 | ↑ | ↑ | ↑ |
| 499 | | ↑ | ↑ | 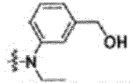 |
| 500 | ↑ | ↑ | ↑ | 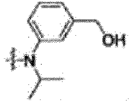 |
| 601 | ↑ | ↑ | ↑ | 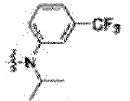 |
| 502 | ↑ | ↑ | ↑ | 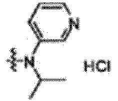 |
| 503 | ↑ | ↑ | ↑ | 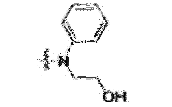 |
| 504 | ↑ | ↑ | ↑ | 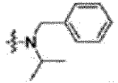 |
| 505 | ↑ | ↑ | ↑ | 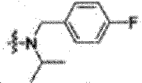 |
| 506 | ↑ | ↑ | ↑ | 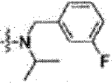 |
| 507 | ↑ | ↑ | ↑ | 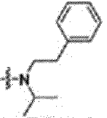 |
| 508 | ↑ | ↑ | ↑ | 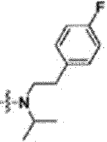 |
| 509 | 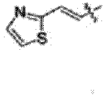 | ↑ | ↑ | 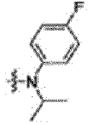 |
| 510 | 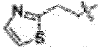 | ↑ | ↑ | ↑ |
| 511 | 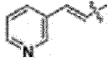 | ↑ | ↑ | ↑ |
| 612 | 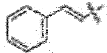 | 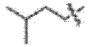 | ↑ | ↑ |
| 513 | ↑ | 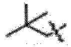 | ↑ | ↑ |
| 514 | ↑ | 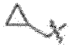 | ↑ | 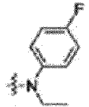 |
| 515 | ↑ | ↑ | ↑ | 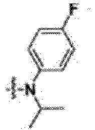 |
| 516 | ↑ | ↑ (Optically active form) | ↑ | ↑ |
| 517 | 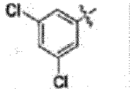 | 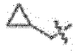 | ↑ | ↑ |
| 518 | 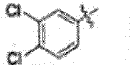 | ↑ | ↑ | ↑ |
| 519 | 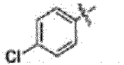 | ↑ | ↑ | ↑ |
| 530 | 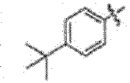 | ↑ | ↑ | ↑ |
| 521 | 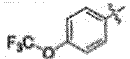 | ↑ | ↑ | ↑ |
| 522 | 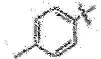 | ↑ | ↑ | ↑ |
| 523 | 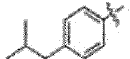 | ↑ | ↑ | ↑ |
| 524 | 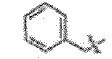 | ↑ | ↑ | ↑ |
| 525 | 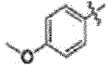 | ↑ | ↑ | ↑ |
| 526 | 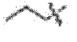 | ↑ | ↑ | ↑ |
| 527 | 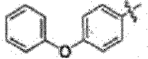 | ↑ | ↑ | ↑ |
| 528 | 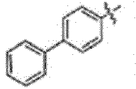 | ↑ | ↑ | ↑ |
| 529 | 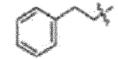 | ↑ | ↑ | ↑ |
| 530 | 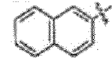 | ↑ | ↑ | ↑ |
| 531 | 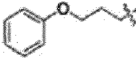 | ↑ | ↑ | ↑ |
| 532 | 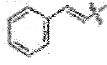 | ↑ | ↑ | 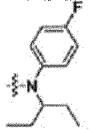 |
| 533 | ↑ | ↑ | ↑ | 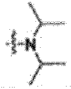 |
| 534 | ↑ | ↑ | ↑ | 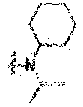 |
| 535 | 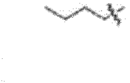 | ↑ | ↑ | 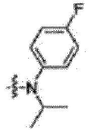 |
| 536 | 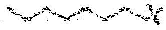 | ↑ | ↑ | ↑ |
| 537 | 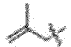 | ↑ | ↑ | ↑ |
| 538 | 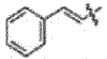 | 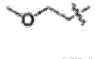 | ↑ | ↑ |
| 539 | ↑ | 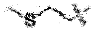 | ↑ | ↑ |
| 540 | ↑ | 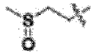 | ↑ | ↑ |
| 541 | ↑ | 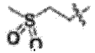 | ↑ | ↑ |
| 542 | ↑ | 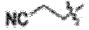 | ↑ | 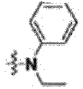 |
| 543 | 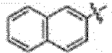 | ↑ | ↑ | ↑ |
| 544 | 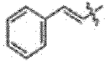 | ↑ | ↑ | 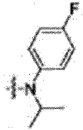 |
| 545 | 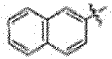 | 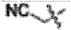 | ↑ | 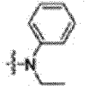 |
| 546 | 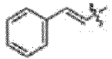 | ↑ | ↑ | ↑ |
| 547 | ↑ | 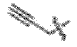 | ↑ | 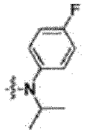 |
| 548 | ↑ | 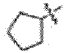 | ↑ | ↑ |
| 549 | 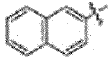 | 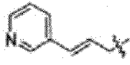 | ↑ | 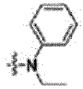 |
| 550 | ↑ | 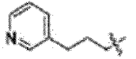 | ↑ | ↑ |
| 551 | ↑ | 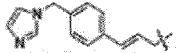 | ↑ | ↑ |
| 552 | ↑ | 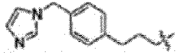 | ↑ | ↑ |
| 553 | ↑ | 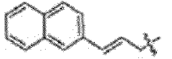 | ↑ | ↑ |
| 554 | ↑ | 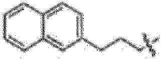 | ↑ | ↑ |
| 555 | ↑ | 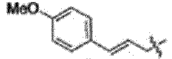 | ↑ | ↑ |
| 556 | ↑ | 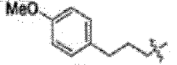 | ↑ | ↑ |
| 557 | ↑ | 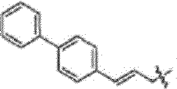 | ↑ | ↑ |
| 558 | ↑ | 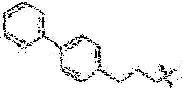 | ↑ | ↑ |
| 559 | ↑ | 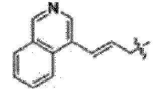 | ↑ | ↑ |
| 560 | ↑ | 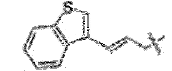 | ↑ | ↑ |
| 561 | ↑ | 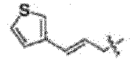 | ↑ | ↑ |
| 562 | ↑ | 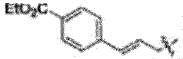 | ↑ | ↑ |
| 563 | ↑ | 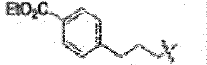 | ↑ | ↑ |
| 564 | ↑ | 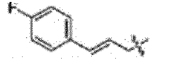 | ↑ | ↑ |
| 565 | ↑ | 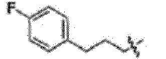 | ↑ | ↑ |
| 566 | ↑ | 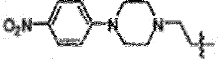 | ↑ | ↑ |
| 567 | ↑ | 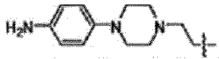 | ↑ | ↑ |
| 568 | 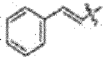 | F, F | | 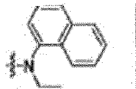 |
| 569 | ↑ | Me, Me | | 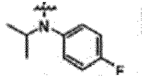 |
| 570 | ↑ | 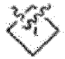 | | 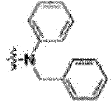 |
| 571 | ↑ | ↑ | | 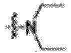 |
| 572 | ↑ | ↑ | | 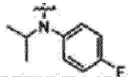 |
| 573 | ↑ | 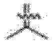 | | ↑ |
| 574 | 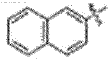 | 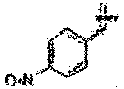 | | 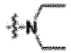 |
| 575 | ↑ | 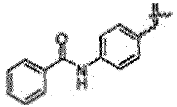 | | ↑ |
| 576 | ↑ | 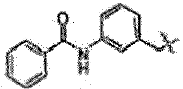 | H | ↑ |
| 577 | ↑ | 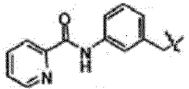 | ↑ | ↑ |
| 578 | ↑ | 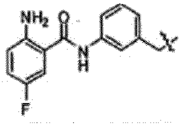 | ↑ | ↑ |
| 579 | ↑ | 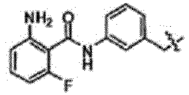 | ↑ | ↑ |
| 580 | ↑ | 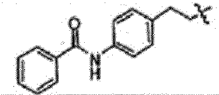 | ↑ | ↑ |
| 581 | ↑ | 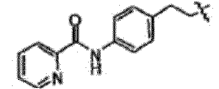 | ↑ | ↑ |
| 582 | ↑ | 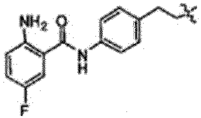 | ↑ | ↑ |
| 583 | ↑ | 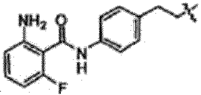 | ↑ | ↑ |
| 584 | ↑ | 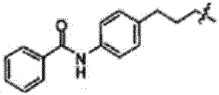 | ↑ | ↑ |
| 585 | ↑ | 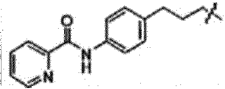 | ↑ | ↑ |
| 586 | ↑ | 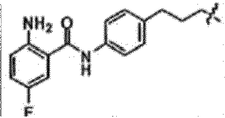 | ↑ | ↑ |
| 587 | ↑ | 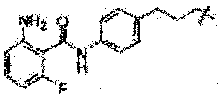 | ↑ | ↑ |
| 588 | 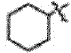 | 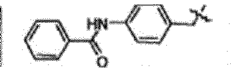 | ↑ | ↑ |

Particularly preferred AT₂ receptor agonists as an active ingredient of the present invention are the following compounds or pharmacologically acceptable salts thereof:
N,N-diethyl-2-{4-[(2,6-difluorobenzoyl)amino]benzyl}-N'-(2-naphthylsulfonyl)malonamide,
(2S)-2-[4-(benzoylamino)benzyl]-N,N-diethyl-N'-(2-naphthyls ulfonyl)malonamide,
(2S)-N,N-diethyl-2-{4-[(2-fluorobenzoyl)amino]benzyl}-N'-(2 -naphthylsulfonyl)malonamide,
(2S)-N,N-diethyl-2-{4-[(3-fluorobenzoyl)amino]benzyl}-N'-(2 -naphthylsulfonyl)malonamide,
(2S)-N,N-diethyl-2-{4-[(2,4-difluorobenzoyl)amino]benzyl}-N '-(2-naphthylsulfonyl)malonamide,
(2S)-N,N-diethyl-2-{4-[(4-methylbenzoyl)amino]benzyl}-N'-(2 -naphthylsulfonyl)malonamide,
(2S)-N,N-diethyl-N'-(2-naphthylsulfonyl)-2-{4-[(2-thienoyl) amino]benzyl}malonamide,
(2S)-N,N-diethyl-2-{4-[(2-furoyl)amino]benzyl}-N'-(2-naphth ylsulfonyl)malonamide,
(2S)-2-{4-[(2-amino-5-fluorobenzoyl)amino]benzyl}-N,N-dieth yl-N'-(2-naphthylsulfonyl)malonamide,
(2S)-2-{4-[(2-amino-6-fluorobenzoyl)amino]benzyl}-N,N-dieth yl-N'-(2-naphthylsulfonyl)malonamide,
(2S)-N,N-diethyl-N'-(2-naphthylsulfonyl)-2-{4-[(2-pyridylca rbonyl)amino]benzyl}malonamide,
(2S)-2-{4-[(2-amino-4-chlorobenzoyl)amino]benzyl}-N,N-dieth yl-N'-(2-naphthylsulfonyl)malonamide,
(2S)-2-{4-[(2-aminobenzoyl)amino]benzyl}-N,N-diethyl-N'-(2-naphthylsulfonyl)malonamide,
(2S)-2-{4-[(2-amino-5-chlorobenzoyl)amino]benzyl}-N,N-dieth yl-N'-(2-naphthylsulfonyl)malonamide,
(2S)-2-{4-[(2-amino-4,5-difluorobenzoyl)amino]benzyl}-N,N-d iethyl-N'-(2-naphthylsulfonyl)malonamide,
(2S)-2-{4-[(2-amino-4-fluorobenzoyl)amino]benzyl}-N,N-dieth yl-N'-(2-naphthylsulfonyl)malonamide,
(2S)-2-{4-[(2-amino-5-methylbenzoyl)amino]benzyl}-N,N-dieth yl-N'-(2-naphthylsulfonyl)malonamide,
2-(4-fluorobenzyl)-N-isopropyl-N-(3-pyridyl)-N'-((E)-styryl sulfonyl)malonamide,
2-allyl-N-(4-fluorophenyl)-N-isopropyl-N'-((E)-styrylsulfon yl)malonamide,
N-(4-fluorophenyl)-2-isobutyl-N-isopropyl-N'-((E)-styrylsul fonyl)malonamide,
N-(4-fluorophenyl)-2-isobutyl-N-isopropyl-N'-phenethylsulfo nyl malonamide,
N-(4-fluorophenyl)-2-isobutyl-N-isopropyl-N'-(2-naphthylsul fonyl)malonamide,
(2S or 2R)-2-cyclopropylmethyl-N-(4-fluorophenyl)-N-isopropyl-N'-( (E)-2-styrylsulfonyl)malonamide,
2-cyclopropylmethyl-N-(4-fluorophenyl)-N-isopropyl-N'-phene thylsulfonyl malonamide, or
2-cyclopropylmethyl-N-(4-fluorophenyl)-N-isopropyl-N'-(2-na phthylsulfonyl)malonamide.

Examples of the pharmacologically acceptable salt of the AT₂ receptor agonist of the present invention include inorganic acid addition salts (for example, salts with hydrochloric acid, hydrobromic acid, hydroiodic acid, sulfuric acid, phosphoric acid, etc.), organic acid addition salts (for example, salts with methanesulfonic acid, benzenesulfonic acid, p-toluenesulfonic acid, formic acid, acetic acid, trifluoroacetic acid, oxalic acid, citric acid, malonic acid, fumaric acid, glutaric acid, adipic acid, maleic acid, tartaric acid, succinic acid, mandelic acid, malic acid, pantothenic acid, methylsulfuric acid, etc.), inorganic base addition salts (for example, salts with sodium, potassium, calcium, magnesium, etc.), salts with amino acids (for example, salts with glutamic acid, aspartic acid, arginine, lysine, etc.), etc.

The AT₂ receptor agonist or a pharmacologically acceptable salt thereof used in the present invention can exhibit polymorphism and can have optical isomers or stereoisomers when having an asymmetric carbon in the molecule. The present invention also encompasses hydrates and solvates thereof. In addition, depending on the presence of an unsaturated bond, the type of substituent(s), pH, etc., more than one tautomers can exist. Therefore, the present invention encompasses the stereoisomers, optical isomers, polymorphs, and tautomers mentioned above, and mixtures thereof, etc.

The AT₂ receptor agonist or a pharmacologically acceptable salt thereof used in the present invention can be produced by a publicly known method. For example, the compound of the general formula (I) or a pharmacologically acceptable salt thereof can be produced by the method described in WO 2008/156142.

The medicament of the present invention is not particularly limited as long as it comprises the above-mentioned AT₂ receptor agonist or a pharmacologically acceptable salt thereof as an active ingredient. The medicament may further comprise a publicly known pharmacologically acceptable inert carrier, excipient, diluent, etc. In the method for preventing or inhibiting acute kidney injury, a medicament comprising the AT₂ receptor agonist is preferably administered to a patient.

The dosage of the medicament of the present invention varies with the administration route, the target disease, and the symptoms, body weight, age, etc. of the patient, and can be appropriately adjusted depending on the purpose of the administration. Usually, the dosage for oral administration to an adult human is 0.01 to 1000 mg/kg body weight per day, preferably 0.05 to 500 mg/kg body weight per day in terms of the amount of the AT₂ receptor agonist, and an appropriately determined dosage may be administered once daily or in several divided doses.

The medicament, the prevention or inhibition method, etc. of the present invention more effectively prevent or inhibit acute kidney injury associated with the treatment of a malignant tumor when the administration to the patient is performed before or during (preferably before) the administration of at least one anticancer and/or antitumor agent that can cause acute kidney injury (in particular, a platinum-based antitumor agent, such as cisplatin).

The medicament of the present invention can be separately administered from said at least one anticancer/antitumor agent (for example, a platinum-based antitumor agent, such as cisplatin). The dosage form and the administration route of the medicament of the present invention are not particularly limited, and depending on the conditions of the patient, one or more oral or parenteral agents may be selected. The duration of the administration of the medicament of the present invention may be appropriately determined depending on the administration method.

The medicament, the treatment or prevention method, etc. of the present invention, when combined with at least one of other usually used anticancer and/or antitumor agents, achieve more effective treatment of a malignant tumor while avoiding the risk of acute kidney injury. The present invention encompasses such a combination treatment with an anticancer agent and/or an antitumor agent. An example of such a combination treatment is an embodiment in which the medicament of the present invention is administered to a patient before or during (preferably before) the administration of an anticancer agent and/or an antitumor agent. By appropriately administering the medicament of the present invention before or during a treatment using an anticancer agent and/or an antitumor agent, the efficiency of the treatment with the anticancer agent and/or the antitumor agent and the prognosis of the patient are improved.

By using the medicament of the present invention together with at least one of usually used anticancer and/or antitumor agents, efficient treatment of a malignant tumor with the anticancer agent and/or the antitumor agent is achieved.

The present invention also encompasses a therapeutic agent for malignant tumors comprising, as active ingredients, the medicament and an anticancer agent and/or an antitumor agent.

The present invention also encompasses a therapeutic agent for malignant tumors comprising the medicament, the therapeutic agent being administered in combination with an anticancer agent and/or an antitumor agent. The present invention also encompasses a therapeutic agent for malignant tumors comprising an anticancer agent and/or an antitumor agent, the therapeutic agent being administered in combination with the medicament.

The present invention also encompasses use of the medicament for producing a therapeutic medicament for malignant tumors associated with an anticancer agent and/or an antitumor agent.

The present invention also encompasses a method for treating malignant tumors associated with an anticancer agent and/or an antitumor agent, the method comprising administering the medicament to a patient.

In the cases where the medicament of the present invention is administered (in combination or together) with an anticancer agent and/or an antitumor agent, the dosage of the anticancer agent and/or the antitumor agent is not particularly limited and can be determined as appropriate depending on the type of the agent, the type of disease (malignant tumor); the age, body weight, and severity of the symptoms of the individual (patient); and the route of administration. The dosage may be a usual dosage.

In the cases where the medicament of the present invention is administered (in combination) together with an anticancer agent and/or an antitumor agent, the dosage of the medicament of the present invention varies with the type of disease (malignant tumor); the age, body weight, and severity of the symptoms of the individual (patient); and the route of administration, and can be appropriately selected. The AT₂ receptor agonist is preferably administered (for example, orally administered) in an appropriately determined dosage, for example, about 0.01 to 1000 mg/kg body weight per day, preferably 0.05 to 500 mg/kg body weight per day once daily or in several divided doses.

In the cases where the medicament of the present invention is used together with an anticancer agent and/or an antitumor agent, the medicament is preferably administered before (or prior to) the administration of the anticancer agent and/or the antitumor agent. The medicament of the present invention is only required to be administered at least before the administration of an anticancer agent and/or an antitumor agent, and may be continuously administered for the required treatment duration.

Usually, the administration of the medicament of the present invention may be started about 1 or more days (for example, 3 or more days), preferably 1 week or more, still more preferably about 10 days or more before the start of the administration of the anticancer agent and/or the antitumor agent. Preferably, the administration of the medicament of the present invention is continued for about 1 or more days (for example, 3 or more days), preferably 1 week or more, preferably about 10 days or more after the end of the administration of the anticancer agent and/or the antitumor agent.

In the cases where an anticancer agent and/or an antitumor agent (for example, a platinum-based antitumor agent, such as cisplatin) is administered, the medicament of the present invention may be provided as one or more kinds of oral or parenteral dosage forms appropriately selected depending on the conditions of the patient, and two or more kinds of oral and parenteral dosage forms can be used in combination.

In the cases where an anticancer agent and/or an antitumor agent (for example, a platinum-based antitumor agent, such as cisplatin) is administered, the medicament of the present invention can be administered at the same time or at a different time.

In such combined administration, the administration of the medicament of the present invention is usually started about 1 week or more, preferably about 10 days or more before the start of the administration of the anticancer/antitumor agent, and continued for about 1 week or more, preferably about 10 days or more after the end of the administration of the anticancer/antitumor agent.

The dosage form and the administration route of the medicament of the present invention in such combined administration are not particularly limited, and one or more kinds of oral or parenteral dosage forms can be selected depending on the conditions of the patient. One or more of oral dosage forms and one or more of parenteral dosage forms can be used in combination.

Examples of the anticancer agent and the antitumor agent used in combination with the medicament of the present invention include an alkylating agent, an antimetabolite, an antitumor antibiotic, an antitumor plant constituent, a BRM (biological response modifier), a hormone, a vitamin, an antitumor antibody, a molecular target drug, a platinum-based antitumor agent, and other anticancer agents and antitumor agents. Among them, preferred as the anticancer agent and the antitumor agent used in combination with the medicament of the present invention is a platinum-based antitumor agent.

More specifically, examples of the alkylating agent include alkylating agents, such as nitrogen mustard, nitrogen mustard N-oxide and chlorambucil; aziridine alkylating agents, such as carboquone and thiotepa; epoxide alkylating agents, such as dibromomannitol and dibromodulcitol; nitrosourea alkylating agents, such as carmustine, lomustine, semustine, nimustine hydrochloride, streptozocin, chlorozotocin, and ranimustine; busulfan, improsulfan tosilate, dacarbazine, etc.

Examples of various antimetabolites include purine antimetabolites, such as 6-mercaptopurine, 6-thioguanine, and thioinosine; pyrimidine antimetabolites, such as fluorouracil, tegafur, tegafur-uracil, carmofur, doxifluridine, broxuridine, cytarabine, and enocitabine; folate antimetabolites, such as methotrexate and trimetrexate; etc.

Examples of the antitumor antibiotic include anthracycline antibiotic antitumor agents, such as mitomycin-C, bleomycin, peplomycin, daunorubicin, aclarubicin, doxorubicin, pirarubicin, THP-adriamycin, 4'-epidoxorubicin, and epirubicin; chromomycin A3; actinomycin-D; etc.

Examples of the antitumor plant constituent include vinca alkaloids, such as vindesine, vincristine, and vinblastine; taxanes, such as paclitaxel and docetaxel; epipodophyllotoxins, such as etoposide and teniposide; etc.

Examples of the BRM include a tumor necrosis factor, indomethacin, etc.

Examples of the hormone include hydrocortisone, dexamethasone, methylprednisolone, prednisolone, prasterone, betamethasone, triamcinolone, oxymetholone, nandrolone, methenolone, fosfestrol, ethinylestradiol, chlormadinone, medroxyprogesterone, etc.

Examples of the vitamin include vitamin C and vitamin A.

Examples of the antitumor antibody and the molecular target drug include trastuzumab, rituximab, cetuximab, nimotuzumab, denosumab, bevacizumab, infliximab, imatinib mesylate, gefitinib, erlotinib, sunitinib, lapatinib, sorafenib, etc.

Examples of the platinum-based antitumor agent include cisplatin, carboplatin, oxaliplatin, etc. Among them, cisplatin is preferred.

Examples of other anticancer agents or antitumor agents include tamoxifen, camptothecin, ifosfamide, cyclophosphamide, melphalan, L-asparaginase, aceglatone, sizofiran, picibanil, procarbazine, pipobroman, neocarzinostatin, hydroxyurea, ubenimex, krestin, etc.

In the cases where the medicament of the present invention is administered in combination with an anticancer agent and/or an antitumor agent, the medicament and the antitumor agent and/or the antitumor agent as active ingredients may be contained in a single formulation or in different formulations.

In the cases where the medicament of the present invention is used with an anticancer agent and/or an antitumor agent, the medicament and the anticancer agent and/or the antitumor agent may be used in any combination. The platinum-based antitumor agent to be used in combination with the medicament of the present invention is preferably cisplatin.

In the present invention, "combined administration" of multiple active ingredients or drugs means that a subject to receive the administration takes all the combined active ingredients or drugs into the body in a certain period of time. The active ingredients may be administered as a single formulation containing all the ingredients (so-called a combination medicine) . Alternatively, the active ingredients may be separately formulated into separate formulations and then separately administered (so-called combined administration) . In the cases where the active ingredients are separately formulated, the timing of the administration is not particularly limited. The formulations may be administered simultaneously, or administered on the same day at certain time intervals, or administered on different days. In the cases where two or more active ingredients are administered at different timings on the same day or administered on different days, the order of administration of the active ingredients is not particularly limited. Normally, each formulation is administered according to each administration method, and therefore the frequency of the administration may be the same or different among the formulations. In the cases where each active ingredient is separately formulated, the administration method (route of administration) may be the same or different among the formulations. It is not necessary that all the active ingredients are present in the body at the same time. As long as all the active ingredients are taken into the body during a certain period of time (for example, one month, preferably one week, more preferably several days, still more preferably one day), it is allowable that one active ingredient has already disappeared from the body when another active ingredient is administered.

### EXAMPLES

Hereinafter, the invention will be specifically described by referring to the Examples below. The Examples are merely illustrative examples of the embodiments of the present invention, and the present invention is not limited thereto.

The experimental materials used in the Examples below were obtained and prepared as follows.

The cisplatin (CDDP) used was cis-diammineplatinum(II) dichloride (Sigma) or CISPLATIN Injection (Nichi-Iko Pharmaceutical). The urea nitrogen measurement kit used was DRI-CHEM (FUJI DRI-CHEM SLIDE BUN-PIII, Fujifilm). The blood creatinine measurement kit used was DRI-CHEM (FUJI DRI-CHEM SLIDE CRE-PIII, Fujifilm) . The urinary creatinine measurement kit used was a chemical assay kit (The Creatinine Companion, Exocell) . The urinary albumin measurement kit used was an ELISA kit (Albuwell M, Exocell).

The AT₂ receptor agonists used were the compounds shown below.
Compound A: N-(4-fluorophenyl)-2-isobutyl-N-isopropyl-N'-((E)-styrylsul fonyl)malonamide
Compound B: (2S)-2-{4-[(2-amino-4-chlorobenzoyl)amino]benzyl}-N,N-dieth yl-N'-(2-naphthylsulfonyl)malonamide
Compound C: (2S)-2-[4-(benzoylamino)benzyl]-N,N-diethyl-N'-(2-naphthyls ulfonyl)malonamide

### Example 1

### Acute kidney injury-inhibiting effects in CDDP induced acute kidney injury model

To 8- to 9-week-old male C57BL/6 mice (n = 7 to 14), 15 mg/kg of cisplatin (CDDP, disease group) or 15 mL/kg of phosphate buffered saline (PBS, normal group) was intraperitoneally administered. A 0.2 mg/mL aqueous solution of Compound A, B or C in 0.5% carboxymethylcellulose (CMC) was prepared, and each solution was orally administered so that Compounds A and C were at a dose of 30 mg/kg per day and Compound B was at a dose of 45 mg/kg per day from 3 days before the start of the experiment, and the mice were subjected to the experiment. To the control group for the comparison with Compounds A, B, and C, the same dose of 0.5% CMC aqueous solution was administered. At 72 hours after CDDP administration, incision in the abdomen was performed under sevoflurane anesthesia, and the whole blood was collected from the abdominal great vein using a syringe. The blood was centrifuged, and the supernatant (serum) was collected. The serum was stored at -20 to -80°C. Immediately before dissection, spot urine was collected from each mouse.

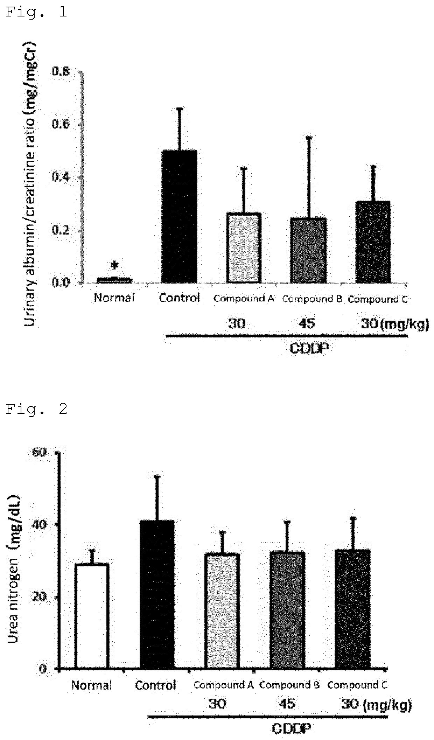

Using the measurement kits, blood urea nitrogen (BUN), serum creatinine (Cr), urinary albumin (Alb), and urinary creatinine (Cr) were measured, and the urinary Alb/Cr ratio was calculated. The results are shown in Fig. 1 and Fig. 2.

Figs. 1 and 2 show that the administration of cisplatin (CDDP) caused acute kidney injury, but Compounds A to C significantly inhibited the acute kidney injury caused by CDDP.

### Example 2

### CDDP induced acute kidney injury-inhibiting effects on AT₂ receptor deficient mice

To 8- to 12-week-old male AT₂ receptor deficient mice (n = 3 or 5 to 7), 15 mg/kg of cisplatin (CDDP, disease group) or 30 mL/kg of phosphate buffered saline (PBS, normal group) was intraperitoneally administered.

A 0.2 mg/mL aqueous solution of Compound C in 0.5% carboxymethylcellulose (CMC) was prepared and orally administered so that Compound C was at a dose of 30 mg/kg per day from 3 days before the start of the experiment, and the mice were subjected to the experiment. To the control group, the same dose of 0.5% CMC aqueous solution was administered.

At 72 hours after CDDP administration, incision in the abdomen was performed under sevoflurane anesthesia, and the whole blood was collected from the abdominal great vein using a syringe. The blood was centrifuged, and the supernatant (serum) was collected. The serum was stored at -20 to -80°C. Immediately before dissection, spot urine was collected from each mouse.

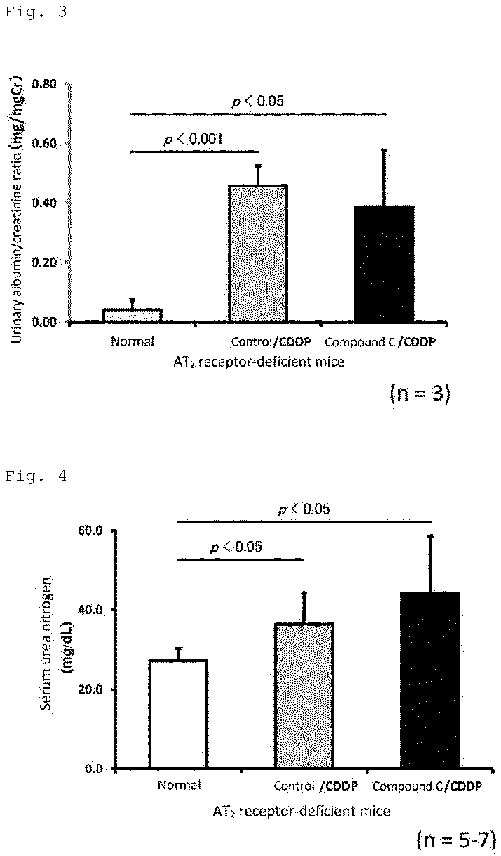

Using the measurement kits, urinary albumin (Alb) and urinary creatinine (Cr) were measured for the calculation of the urinary Alb/Cr ratio, and also blood urea nitrogen (BUN) was measured. The results are shown in Fig. 3 (urinary Alb/Cr ratio) and Fig. 4 (BUN).

Figs. 3 and 4 show that the administration of cisplatin (CDDP) to AT₂ receptor deficient mice caused acute kidney injury, and administration of Compound C was not effective in inhibiting the acute kidney injury caused by CDDP.

The results of Experiment 1 and Experiment 2 suggest that Compound C, which is an AT₂ receptor agonist, acts on the AT₂ receptor itself and thereby inhibits acute kidney injury. Therefore, an AT₂ receptor agonist that acts on AT₂ receptors is capable of preventing or treating acute kidney injury.

### INDUSTRIAL APPLICABILITY

The present invention provides an excellent medicament for preventing or inhibiting acute kidney injury associated with an anticancer agent and/or an antitumor agent, and the medicament is useful in the medical field etc.

## Claims

1. A medicament for preventing or inhibiting acute kidney injury, the medicament comprising, as an active ingredient, an angiotensin II receptor type 2 agonist or a pharmacologically acceptable salt thereof.

2. The medicament according to claim 1, wherein the acute kidney injury is acute kidney injury induced by an anticancer agent and/or an antitumor agent.

3. The medicament according to claim 2, wherein the anticancer agent and/or the antitumor agent is a platinum-based antitumor agent.

4. The medicament according to any one of claims 1 to 3, wherein the angiotensin II receptor type 2 agonist is a sulfonyl malonamide derivative represented by the following general formula (I): 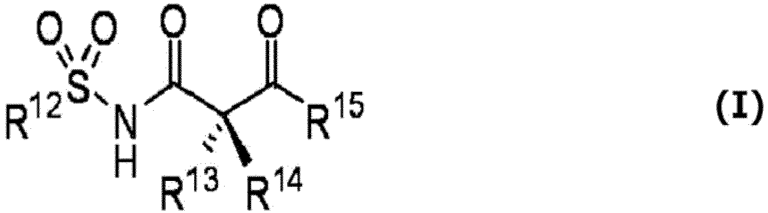 [wherein
R¹² denotes 2-naphthyl, trans-β-styryl, phenethyl, 3-phenoxypropyl, or 4-phenylbutyl;
either of R¹³ and R¹⁴ denotes a hydrogen atom, and the other denotes isopropyl, isobutyl, neopentyl, allyl, -CH₂-R¹⁶ {wherein R¹⁶ denotes optionally substituted C₃₋₁₀ cycloalkyl, an optionally substituted heterocycle, or -CO-NR⁵R⁶ (wherein R⁵ and R⁶ may be the same or different and each represent a hydrogen atom, C₁₋₆ alkyl, optionally substituted aryl, or optionally substituted heteroaryl, or R⁵ and R⁶ may form, together with the nitrogen atom to which they are bonded, optionally substituted cyclic amino)}, -(CH₂)₂-R^{16'} (wherein R^{16'} denotes cyano or C₁₋₆ alkoxy), or -(CH₂)ₙ-Ar² (wherein n denotes an integer of 1 to 3, and Ar² denotes substituted phenyl or optionally substituted heteroaryl), or R¹³ and R¹⁴ may form, together with the carbon atom to which they are bonded, a moiety represented by the following formula: 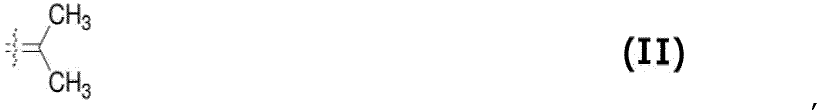 and
R¹⁵ denotes di(C₁₋₆ alkyl)amino or a moiety represented by the following formula: 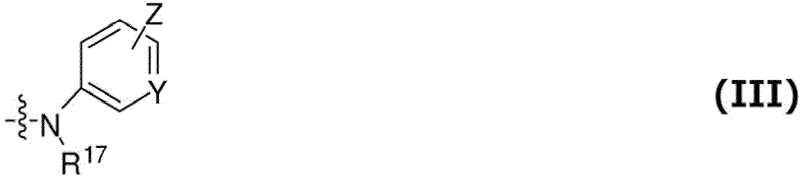 (wherein Z denotes a hydrogen atom, a halogen atom, or trifluoromethyl, Y denotes a nitrogen atom or CH, and R¹⁷ denotes ethyl, isopropyl, or 3-pentyl, with the proviso that when Y is a nitrogen atom, Z denotes a hydrogen atom)], or a pharmacologically acceptable salt thereof.

5. The medicament according to claim 4, wherein the angiotensin II receptor type 2 agonist is a sulfonyl malonamide derivative selected from
N,N-diethyl-2-{4-[(2,6-difluorobenzoyl)amino]benzyl}-N'-(2-naphthylsulfonyl)malonamide,
(2S)-2-[4-(benzoylamino)benzyl]-N,N-diethyl-N'-(2-naphthyls ulfonyl)malonamide,
(2S)-N,N-diethyl-2-{4-[(2-fluorobenzoyl)amino]benzyl}-N'-(2 -naphthylsulfonyl)malonamide,
(2S)-N,N-diethyl-2-{4-[(3-fluorobenzoyl)amino]benzyl}-N'-(2 -naphthylsulfonyl)malonamide,
(2S)-N,N-diethyl-2-{4-[(2,4-difluorobenzoyl)amino]benzyl}-N '-(2-naphthylsulfonyl)malonamide,
(2S)-N,N-diethyl-2-{4-[(4-methylbenzoyl)amino]benzyl}-N'-(2 -naphthylsulfonyl)malonamide,
(2S)-N,N-diethyl-N'-(2-naphthylsulfonyl)-2-{4-[(2-thieno yl)amino]benzyl}malonamide,
(2S)-N,N-diethyl-2-{4-[(2-furoyl)amino]benzyl}-N'-(2-naphth ylsulfonyl)malonamide,
(2S)-2-{4-[(2-amino-5-fluorobenzoyl)amino]benzyl}-N,N-dieth yl-N'-(2-naphthylsulfonyl)malonamide,
(2S)-2-{4-[(2-amino-6-fluorobenzoyl)amino]benzyl}-N,N-dieth yl-N'-(2-naphthylsulfonyl)malonamide,
(2S)-N,N-diethyl-N'-(2-naphthylsulfonyl)-2-{4-[(2-pyridylca rbonyl)amino]benzyl}malonamide,
(2S)-2-{4-[(2-amino-4-chlorobenzoyl)amino]benzyl}-N,N-dieth yl-N'-(2-naphthylsulfonyl)malonamide,
(2S)-2-{4-[(2-aminobenzoyl)amino]benzyl}-N,N-diethyl-N'-(2-naphthylsulfonyl)malonamide,
(2S)-2-{4-[(2-amino-5-chlorobenzoyl)amino]benzyl}-N,N-dieth yl-N'-(2-naphthylsulfonyl)malonamide,
(2S)-2-{4-[(2-amino-4,5-difluorobenzoyl)amino]benzyl}-N,N-d iethyl-N'-(2-naphthylsulfonyl)malonamide,
(2S)-2-{4-[(2-amino-4-fluorobenzoyl)amino]benzyl}-N,N-dieth yl-N'-(2-naphthylsulfonyl)malonamide,
(2S)-2-{4-[(2-amino-5-methylbenzoyl)amino]benzyl}-N,N-dieth yl-N'-(2-naphthylsulfonyl)malonamide,
2-(4-fluorobenzyl)-N-isopropyl-N-(3-pyridyl)-N'-((E)-styryl sulfonyl)malonamide,
2-allyl-N-(4-fluorophenyl)-N-isopropyl-N'-((E)-styrylsulfon yl)malonamide,
N-(4-fluorophenyl)-2-isobutyl-N-isopropyl-N'-((E)-styrylsul fonyl)malonamide,
N-(4-fluorophenyl)-2-isobutyl-N-isopropyl-N'-phenethylsulfo nyl malonamide,
N-(4-fluorophenyl)-2-isobutyl-N-isopropyl-N'-(2-naphthylsul fonyl)malonamide,
(2S or 2R)-2-cyclopropylmethyl-N-(4-fluorophenyl)-N-isopropyl-N'-( (E)-2-styrylsulfonyl)malonamide,
2-cyclopropylmethyl-N-(4-fluorophenyl)-N-isopropyl-N'-phene thylsulfonyl malonamide, and
2-cyclopropylmethyl-N-(4-fluorophenyl)-N-isopropyl-N'-(2-na phthylsulfonyl)malonamide, or a pharmacologically acceptable salt thereof.

6. A combination medicament of an angiotensin II receptor type 2 agonist or a pharmacologically acceptable salt thereof and an anticancer agent and/or an antitumor agent.

7. A medicinal composition for preventing or inhibiting acute kidney injury, the medicinal composition comprising, as active ingredients, an angiotensin II receptor type 2 agonist or a pharmacologically acceptable salt thereof and an anticancer agent and/or an antitumor agent.

8. A medicinal composition for preventing or inhibiting acute kidney injury, the medicinal composition comprising an angiotensin II receptor type 2 agonist or a pharmacologically acceptable salt thereof, which composition is used in combination with an anticancer agent and/or an antitumor agent.

9. A method for preventing or inhibiting acute kidney injury, the method comprising administering, to a patient, an effective amount of an angiotensin II receptor type 2 agonist or a pharmacologically acceptable salt thereof in combination with an anticancer agent and/or an antitumor agent.

10. A method for preventing or inhibiting acute kidney injury, the method comprising administering, to a patient, an effective amount of an angiotensin II receptor type 2 agonist or a pharmacologically acceptable salt thereof.

11. The method according to claim 10, wherein the acute kidney injury is acute kidney injury induced by an anticancer agent and/or an antitumor agent.

12. An angiotensin II receptor type 2 agonist or a pharmacologically acceptable salt thereof for use in preventing or inhibiting acute kidney injury.

13. The angiotensin II receptor type 2 agonist or a pharmacologically acceptable salt thereof for use according to claim 12, wherein the acute kidney injury is acute kidney injury induced by an anticancer agent and/or an antitumor agent.

14. Use of an angiotensin II receptor type 2 agonist or a pharmacologically acceptable salt thereof for producing a medicament for preventing or inhibiting acute kidney injury.

15. The use according to claim 14, wherein the acute kidney injury is acute kidney injury induced by an anticancer agent and/or an antitumor agent.

16. Use of an angiotensin II receptor type 2 agonist or a pharmacologically acceptable salt thereof for preventing or inhibiting acute kidney injury.

17. The use according to claim 16, wherein the acute kidney injury is acute kidney injury induced by an anticancer agent and/or an antitumor agent.
